# EUROPEAN PATENT APPLICATION

(11) **EP 4 094 572 A1**
(43) Date of publication of application: **30.11.2022**
(21) Application number: 21305709.4
(22) Date of filing: 28.05.2021
(51) Int. Cl.: A01H 1/04, A01H 6/46, C12Q 1/6876, C12Q 1/6895

(54) **WHEAT COMPRISING MALE FERTILITY RESTORER ALLELES**

(71) Applicant: Limagrain Europe, 63360 Saint-Beauzire (FR); Biogemma, 63720 Chappes (FR)
(72) Inventor: VARENNE, Pierrick, 77300 FONTAINEBLEAU (FR); THROUDE, Mickaël, 63116 BEAUREGARD L'EVEQUE (FR); SPECEL, Sébastien, 63260 MONTPENSIER (FR); DERORY, Jeremy, 63130 ROYAT (FR); COMADRAN, Jordi, 63200 RIOM (FR)
(74) Representative: Plasseraud IP

(57) **Abstract**

The invention is in the field of plant genetics and plant breeding. The invention more specifically relates to wheat plants carrying restorer of fertility genes specific to *T. timopheevii* CMS cytoplasm, in particular restorer alleles Rf1, Rf3, Rf4s, Rf7 and 6R.

## Description

The invention is in the field of plant genetics and plant breeding. The invention more specifically relates to wheat plants carrying restorer of fertility genes specific to *T. timopheevii* CMS cytoplasm.

### BACKGROUND

Hybrid production is based on crossing two parental lines to beneficiate of heterosis and *de facto,* increase genetic variability to create new varieties or genotypes with higher yield and better adapted to environmental stresses. Even in a predominantly autogamous species like wheat, research studies have shown that hybrid lines exhibit improved quality and greater tolerance to environmental and biotic stresses.

In order to promote commercially viable rates of hybrid production, self-fertilization must be avoided, i.e. fertilization of the female organ by the pollen of the same plant. It is desired that the female organ of the female parent is exclusively fertilized with the pollen of the male parent.

Male sterility can be achieved by three different ways. Manual emasculation is the simplest one and is still used in some species where male and female flowers are separated, e.g. corn. However, it is impractical in species like wheat where flowers contain both female and male organs. Male sterility can be induced by chemical hybridization agents (CHAs) with gametocidic effects. Currently, only a few commercial hybrid wheat cultivars are based on this technology as it can bear substantial financial risks.

Finally, male sterility can also be induced by genetic means. There are many examples of hybrid systems in corn or sorghum based on male sterility induced by genetic means showing the preponderance of this technology compared to the two mentioned previously. However, in other species which are predominantly self-pollinated like wheat, hybrid production is still a challenge (Longin et al., 2012).

A system that has been successfully used for production of hybrid in several crop plants including maize, rice and sorghum is the three-line breeding system based on cytoplasmic male sterility (CMS), a genetically conditioned trait that leads to plant sterility.

In order to obtain a reliable and efficient system for producing seeds needed for hybrid production, one generally needs three essential elements: a means to induce male sterility, a means to propagate the sterility, and a means to restore fertility. For example, a fully genetically based system is composed of a male-sterile line (female parent), a fertile maintainer line (male parent allowing propagation of the male-sterile line), and a fertility restorer line (male parent for hybrid production).

The first case of cytoplasmic male sterility in wheat was observed in 1951 (Kihara, 1951), where it was observed that sterility was caused by incompatibility between the cytoplasm of *Aegilops caudata* L. and the nucleus of *T. aestivum* var. erythrospermum. Subsequently research on *T. timopheevii* cytoplasm showed that this cytoplasm is able to induce sterility in bread wheat (*T. aestivum*) (Wilson and Ross, 1961, Crop Sci, 1: 191-193). *Orf256* was previously identified as a gene specific to the *T. timopheevii* mitochondrial genome (Rathburn and Hedgcoth, 1991; Song and Hedgcoth, 1994) responsive of CMS. This hypothesis was recently overcome by Ian Small and Joanna Melonek who pointed the Orf279 mitochondrial protein to being responsive of T-CMS (WO 2020/161261, Melonek and al., 2021).

It was expected that such a cytoplasm could be used in a hybrid production system. However, major limitations arose from the difficulty in finding a completely dominant and stable fertility restorer gene with no negative side effects (notably on yield).

Fertility restoration of male sterile plants harboring *T. timopheevii* CMS cytoplasm (T-CMS cytoplasm) has been reported and nine major restorer loci (designated as Rf1 to Rf9) have been identified and located approximate within the wheat genome (Shahinnia et al., 2020). One of the most effective restorer loci is Rf3 (Ma and Sorrells, 1995; Kojima et al., 1997; Ahmed et al., 2001; Geyer et al., 2016). Two SNP markers allowed the location of the Rf3 locus within a 2 cM fragment on chromosome 1B (Geyer et al., 2016).

While it is understood that restoration to normal pollen fertility could require two or more Rf loci, it is also well known that modifier loci exist that have either minor effect with low penetrance (Zhou et al., 2005; Stojalowski et al., 2013) or inhibitory effects on fertility, depending on environmental conditions (Wilson et al., 1984). It is not yet understood which combination of genes or loci is needed to complete a full restoration of T-CMS in different genetic backgrounds and environmental conditions.

In this context, the development of technologies that enable a full restoration of pollen fertility is of major importance in wheat. WO2019/086510 discloses wheat plants restorer of fertility with some combinations of restorer loci. However, there is still a need to develop new wheat plants restorer of fertility.

It is therefore the object of the present invention to propose suitable fertility restorer genes in wheat for the development of a hybrid production system useful for the seed industry.

### SUMMARY

A first object of the present disclosure relates to a wheat plant restorer of fertility of *T. timopheevii* CMS cytoplasm comprising at least Rf1, Rf3, and Rf4s restorer of fertility alleles. It also relates to a wheat plant restorer of fertility of *T. timopheevii* CMS cytoplasm, wherein the plant comprises at least Rf1, Rf3, and 6R restorer of fertility alleles.

Another aspect relates to a method of identifying a wheat plant by detecting the presence of at least one restorer allele within one or more of Rf1, Rf3, Rf4s, Rf7 and 6R loci, preferably within the three Rf1, Rf3 and Rf4s loci or within the three Rf1, Rf3 and 6R loci.

The disclosure further relates to a method for producing a wheat hybrid plant comprising the steps of:
- providing a first wheat plant comprising one or two restorer allele selected among Rf1, Rf3 and Rf4s restorer alleles,
- crossing said first wheat plant with a second wheat plant comprising one or two restorer alleles selected among Rf1, Rf3 and Rf4s restorer alleles, wherein Rf1, Rf3 and Rf4s restorer alleles are represented at least once in the panel of restorer alleles provided by the first plant and the second plant,
- collecting the F1 hybrid seed,
- obtaining homozygous plants from the F1 plants,
- detecting the presence of the Rf1, Rf3 and Rf4s restorer alleles in the hybrid seed and/or at each generation.

Further, the disclosure also relates to a method for producing a wheat hybrid plant comprising the steps of:
- providing a first wheat plant comprising one or two restorer allele selected among Rf1, Rf3 and 6R restorer alleles,
- crossing said first wheat plant with a second wheat plant comprising one or two restorer alleles selected among Rf1, Rf3 and 6R restorer alleles, wherein Rf1, Rf3 and 6R restorer alleles are represented at least once in the panel of restorer alleles provided by the first plant and the second plant,
- collecting the F1 hybrid seed,
- obtaining homozygous plants from the F1 plants,
- detecting the presence of the Rf1, Rf3 and 6R restorer alleles in the hybrid seed and/or at each generation.

Yet another aspect of the disclosure relates to a method for producing a wheat hybrid plant comprising the steps of:
- crossing a sterile female comprising the *T. timopheevii* cytoplasm with a fertile male wheat plant according to the disclosure;
- collecting the hybrid seed;
- optionally detecting the presence of *T.timopheevii* cytoplasm, and/or at least three of the Rf locus chosen amongst Rf1, Rf3, Rf4s, Rf7 and 6R in the hybrid seed;
- optionally detecting hybridity level of the hybrid seeds.

### DETAILED DESCRIPTION

### The wheat plant restorer of fertility of T. timopheevi CMS cytoplasm

The inventors have shown that a combination of at least 3 specific fertility restorer alleles enable the obtention of wheat plants with full restoration of fertility of *T. timopheevi* CMS cytoplasm. Particularly, the wheat plant comprises at least a fertility restorer allele A, a fertility restorer allele B and a fertility restorer allele C.

As used herein, a fertility restorer A corresponds to Rf3.

As used herein, a fertility restorer B corresponds to Rf1 or Rf7.

As used herein, a fertility restorer C corresponds to Rf4s or 6R.

Therefore, a first aspect of the present disclosure relates to a wheat plant restorer of fertility of T. *timopheevii* CMS cytoplasm, wherein the wheat plant comprises at least three fertility restorer alleles:
- Rf3, and
- Rf1 and/or Rf7, and
- Rf4s and/or 6R.In specific embodiments, the wheat plant comprises at least the three fertility restorer alleles: Rf3, Rf1, and Rf4s.

In specific embodiments, the wheat plant comprises at least the three fertility restorer alleles: Rf3, Rf1, and 6R.

In specific embodiments, the wheat plant comprises at least the three fertility restorer alleles: Rf3, Rf7, and Rf4s.

In specific embodiments, the wheat plant comprises at least the three fertility restorer alleles: Rf3, Rf7, and 6R.

In another specific embodiments, the wheat plant comprises at least the four fertility restorer alleles: Rf3, Rf1, Rf7, and Rf4s.

In another specific embodiments, the wheat plant comprises at least the four fertility restorer alleles: Rf3, Rf1, Rf4s and 6R.

In another specific embodiments, the wheat plant comprises at least the four fertility restorer alleles: Rf3, Rf1, Rf7, and 6R.

In another specific embodiments, the wheat plant comprises at least the four fertility restorer alleles: Rf3, Rf4s, Rf7, and 6R.

This list of examples is not exhaustive.

As described in WO 2019/086510, the inventors have identified two types of Rf3 restorer of fertility, one with a strong fertility restoration, and another with a weak fertility restoration. Rf3 strong and weak alleles can be associated to these combinations of three or four alleles. The Rf3 weak allele should be preferentially associated in a four allele combination.

Whenever reference to a "plant" or "plants" is made, it is understood that also plant parts (cells, tissues or organs, seed pods, seeds, severed parts such as roots, leaves, flowers, pollen, etc.), progeny of the plants which retain the distinguishing characteristics of the parents (especially, male fertility associated with the claimed Rf nucleic acids), such as seed obtained by selfing or crossing, e.g. hybrid seeds (obtained by crossing two inbred parent plants), hybrid plants and plant parts derived therefrom are encompassed herein, unless otherwise indicated.

As used herein, the term "Rf4s" refers to restorer allele Rf4 from *Aegilops speltoides.*

As used herein, the expression "wheat plant" refers to species of the genus *Triticum* as for example, *T. aestivum, T. aethiopicum, T. araraticum, T. boeoticum, T. carthlicum, T. compactum, T. dicoccoides, T. dicoccon, T. durum, T. ispahanicum, T. karamyschevii, T. macha, T. militinae, T. monococcum, T. polonicum, T. spelta, T. sphaerococcum, T. timopheevii, T. turanicum, T. turgidum, T. urartu, T. vavilovii, T. zhukovskyi Faegi.* Wheat plant also refers to species of the genera *Aegilops* and *Triticale.*

As used herein, the expression "restorer of fertility of *T. timopheevi* CMS cytoplasm" refers to a protein whose expression in a wheat plant comprising *T. timopheevi* CMS cytoplasm contributes to the restoration of the production of pollen in the *Triticum timopheevii* CMS system.

As used herein, the term "allele(s)" means any of one or more alternative forms of a gene at a particular locus. In a diploid, alleles of a given gene are located at a specific location or locus on a chromosome. One allele is present on each chromosome of the pair of homologous chromosomes. The same definition is used for plants bearing a higher level of ploidy like in Triticum gender wherein, for example, *T. aestivum* is an hexaploid plant.

As used herein, the expression "restorer allele of *T. timopheevi* CMS cytoplasm" refers to an allele which contributes to the restoration of the production of pollen in the CMS *Triticum timopheevii* system.

The restoration of pollen fertility may be partial or complete. In particular, the fertility score of F1 wheat plants having CMS-T *timopheevii* cytoplasm (from crosses between CMS female lines and restorer lines) may be calculated by dividing the total number of seeds threshed from a spike by the number of counted spikelets and may be compared with the fertility scores of a panel of control fertile plants, for example elite inbred lines bearing a normal wheat cytoplasm, grown in the same area and under the same agro-environmental conditions. It is preferred that such panels of lines comprise a set of at least 5 elite inbred lines wherein these lines are representative of the area where the fertility test is achieved. Besides, it is preferred that at least 10 spikes from different individual F1 plants be assessed for a given experiment. Examples of pollen fertility tests are described in WO2019/086510.

If the fertility score is not null, then the plant has acquired partial or full restoration of fertility. For each fertility score, a statistical test is calculated to obtain a p-value. Examples of statistical tests are the Anova or mean comparison tests. A p-value below a 5% threshold will indicate that the two distributions are statistically different. Therefore, a significant decrease of the fertility score of the tested wheat plant as compared to the fertility score of the fully fertile control plant is indicative that the F1 plant has not acquired full restoration of fertility (i.e. partial restoration). A similar or higher fertility score is indicative that the F1 plant has acquired full restoration of fertility. In a preferred embodiment, the wheat plant, such as transgenic or genetically engineered wheat plant, according to the present disclosure, has acquired full restoration of fertility.

As used herein, the term "crossing" can refer to a simple X by Y cross, or also to the process of backcrossing, depending on the context.

The loci of the restorer alleles of *T. timopheevi* CMS cytoplasm within Rf1, Rf3 and Rf7 have been previously mapped in the WO2019/086510. The corresponding restorer alleles are designated Rf1, Rf3, and Rf7 restorer alleles and have been described in the art.
Further, it is hereby disclosed the mapping of Rf4s restorer allele and a wheat plant comprising thereof.

In particular, a wheat plant source of the Rf3 restorer allele includes the commercial following lines: Allezy, Altigo, Altamira as also detailed in WO2019/086510. A wheat plant source of the Rf4 restorer allele includes the following lines: R113 or L13.

In specific embodiment, representative alleles of Rf1, Rf3, and Rf4s restorer alleles are provided by the seed sample: NCIMB 43746.

In another specific embodiment, representative alleles of Rf1, Rf3, and 6R restorer alleles are provided by the seed sample: NCIMB 43747.

| | | |
|---|---|---|
| NCIMB 43746 | Triticum aestivum/winter wheat | LGWR20-0485 |
| NCIMB 43747 | Triticum aestivum/winter wheat | LGWR17-0160 |

As used herein, the term "chromosomal interval" designates a contiguous linear span of genomic DNA that resides in planta on a single chromosome. The genetic elements or genes located on a single chromosomal interval are physically linked. The size of a chromosomal interval is not particularly limited. In some aspects, the genetic elements located within a single chromosomal interval are genetically linked, typically with a genetic recombination distance of, for example, less than or equal to 20 cM, or alternatively, less than or equal to 10 cM. That is, two genetic elements within a single chromosomal interval undergo recombination at a frequency of less than or equal to 20% or 10%.

As used herein a "marker" refers to a specific DNA sequence identified within the genome of a plant and which can be used to determine whether a plant has inherited a particular phenotype or allele of interest from a parent plant. Said marker may include coding or non-coding sequences. In particular, said marker may include one or more Single Nucleotide Polymorphism or SNP identified within the plant genome.

As used herein, the Rf1 locus refers to the locus of the Rf1 restorer allele, which locus is located at most 10 cM, preferably at most 7 cM, more preferably at most 2 cM, from marker cfn0522096 of SEQ ID NO:3 and/or from marker cfn05277067 of SEQ ID NO:9. In a specific embodiment, the wheat plant restorer of fertility according to the present disclosure includes at least one Rf1 restorer allele, said Rf1 restorer allele being located within the chromosomal interval between SNP markers cfn0522096 of SEQ ID NO:3 and cfn05277067 of SEQ ID NO:9.

In specific embodiments, the wheat plant restorer of fertility includes one Rf1 restorer allele at the Rf1 locus characterized by the presence of one or more of the SNP allele(s) as identified by Table 1.

**Table 1: SNP markers for mapping of Rf1 locus**

| SNP# | Marker Name | Marker SEQ ID NO: | Restorer Allele |
|---|---|---|---|
| SNP1 | cfn0523109 | 1 | A |
| SNP2 | 276I13_96B22_97797 | 2 | C |
| SNP3 | cfn0522096 | 3 | C |
| SNP4 | cfn0527763 | 4 | C |
| SNP5 | 104A4_105172 | 5 | TG |
| SNP6 | 104A4_105588 | 6 | A |
| SNP7 | cfn0373248 | 7 | T |
| SNP8 | cfn1 097828 | 8 | C |
| SNP9 | cfn0527067 | 9 | A |
| SNP10 | cfn0528390 | 10 | G |
| SNP11 | BWS0267 | 11 | A |
| SNP12 | cfn0527718 | 12 | T |
| SNP13 | cfn0524469 | 13 | G |
| SNP14 | cfn0524921 | 14 | G |
| SNP15 | cfn1122326 | 15 | C |
| SNP16 | RFL79_S7 | 16 | G |

Preferably, the wheat plant restorer of fertility according to the present disclosure includes one Rf1 restorer allele at the Rf1 locus characterized by the presence of the SNP2 and/or SNP6 and/or SNP16 restorer allele(s) as described in Table 1. Preferably, the wheat plant restorer of fertility is characterized by the haplotypes of the SNP2 and SNP6 restorer alleles "C" and "A". More preferably the RF1 restorer allele at the Rf1 locus is SNP16, characterized by the haplotype of the SNP16 restorer allele "G".

In specific embodiments, the wheat plant restorer of fertility with Rf1 restorer allele comprises a Rf1 nucleic acid.

By "Rf1 nucleic acid", it is meant a nucleic acid comprising a gene encoding a Rf1 protein restorer of fertility of *T. timopheevii* CMS cytoplasm, wherein the corresponding amino acid sequence has at least 95% identity, preferably, 96%, 97%, 98%, 99% or 100% identity to SEQ ID NO:64.

In particular, the inventors have previously identified that RFL79 sequence of SEQ ID NO:64 can restore male fertility of CMS-Fielder plants. Accordingly, in a preferred embodiment, examples of Rf1 nucleic acids comprises the disclosed Rf1 nucleic acid sequences of SEQ ID NO:65, SEQ ID NO:66, SEQ ID NO:67, SEQ ID NO:68 or SEQ ID NO:69, preferably a Rf1 nucleic acid comprises SEQ ID NO:69. In these sequences the inventors have identified the marker RFL79_S7 (SNP16). As used herein, the Rf3 locus refers to the locus of the Rf3 restorer allele, which locus is at most 10 cM, preferably at most 7 cM, more preferably at most 2 cM, from marker cfn1249269 of SEQ ID NO:19 and/or from marker BS00090770 of SEQ ID NO:42.

In a specific embodiment, the wheat plant restorer of fertility includes at least one Rf3 restorer allele within the Rf3 locus, said Rf3 restorer allele being located within the chromosomal fragment between SNP markers cfn1249269 and BS00090770.

In another specific embodiment, the wheat plant restorer of fertility includes one Rf3 restorer allele at the Rf3 locus characterized by the presence of one or more of the SNP allele(s) as identified by Table 2.

**Table 2: SNP Markers for mapping of Rf3 locus**

| SNP# | Marker Name | Marker SEQ ID | Restorer Allele |
|---|---|---|---|
| SNP17 | cfn1 252000 | 17 | A |
| SNP18 | IWB14060* | 18 | G |
| SNP19 | cfn1249269 | 19 | G |
| SNP20 | 219K1_166464 | 20 | T |
| SNP21 | 219K1_158251 | 21 | G |
| SNP22 | 219K1_111446 | 22 | A |
| SNP23 | 219K1_110042 | 23 | T |
| SNP24 | 219K1_110005 | 24 | C |
| SNP25 | 219K1_107461 | 25 | A |
| SNP26 | 219K1_99688 | 26 | T |
| SNP27 | 219K1_37 | 27 | C |
| SNP28 | cfn1270524 | 28 | T |
| SNP29 | 136H5_3M5_7601 | 29 | T |
| SNP30 | cfn1288811 | 30 | G |
| SNP31 | 136H5 3M5 89176 | 31 | A |
| SNP32 | 136H5 3M5 89263 | 32 | T |
| SNP33 | 136H5_3M5_138211 | 33 | T |
| SNP34 | cfn0556874 | 34 | C |
| SNP35 | 136H5_3M5_64154 | 35 | C |
| SNP36 | 136H5_3M5_68807 | 36 | G |
| SNP37 | 136H5_3M5_77916 | 37 | A |
| SNP38 | cfn1246088 | 38 | A |
| SNP39 | cfn1287194 | 39 | G |
| SNP40 | cfn1 258380 | 40 | A |
| SNP41 | IWB72107* | 41 | A |
| SNP42 | BS00090770 | 42 | T |
| SNP43 | cfn1 239345 | 43 | A |
| SNP44 | RFL29_S2 | 44 | G |
| SNP 45 | RFL29_S4 | 45 | C |

Preferably, the wheat plant restorer of fertility according to the present disclosure includes one Rf3 restorer allele at the Rf3 locus characterized by the presence of the SNP29 and/or SNP31 restorer allele(s) as described in Table 2. More preferably, the wheat plant restorer of fertility is characterized by the haplotype of the SNP29 and SNP31 restorer alleles "T" and "A" respectively.

In another particular embodiment, that may be combined with the previous embodiments, the wheat plant restorer of fertility according to the present disclosure includes one Rf3 restorer allele at the Rf3 locus characterized by the presence of the SNP38 and SNP41 restorer alleles "A" and "A" respectively.

As described in WO 2019/086510, two types of Rf3 restorer of fertility exist: one with a strong fertility restoration, and another with a weak fertility restoration. In another preferred specific embodiment, the wheat plant restorer of fertility includes one Rf3 strong restorer allele at the Rf3 locus.

The SNP 44 marker, characterized by the restorer allele "G", is a marker of weak Rf3 restorer of fertility allele, the SNP 41 and 45, characterized by restorer alleles "A" and "C" respectively are marker of strong Rf3 restorer of fertility allele.

More preferably, the wheat plant restorer of fertility according to the present disclosure includes one Rf3 restorer allele at the Rf3 locus characterized by the presence of the SNP41 and/or SNP45 restorer allele(s) as described in Table 2. More preferably, the wheat plant restorer of fertility is characterized by the haplotype of the SNP41 and SNP45 restorer alleles "A" and "C" respectively.

In specific embodiments, the wheat plant restorer of fertility with Rf3 restorer allele comprises a Rf3 nucleic acid.

As used herein, the term "Rf3 nucleic acid" refers to a nucleic acid comprising a gene encoding a Rf3 protein restorer of fertility of *T. timopheevii* CMS cytoplasm, wherein the corresponding amino acid sequence has at least 95% identity, preferably, 96%, 97%, 98%, 99% or 100% identity to an amino acid sequence selected from the group consisting of SEQ ID NO:70, SEQ ID NO:71 and SEQ ID NO:72.

Typically, the wheat plant restorer of fertility according to the present disclosure includes Rf3 nucleic acids comprises SEQ ID NO:73, SEQ ID NO:74, SEQ ID NO:75, SEQ ID NO:76, SEQ ID NO:77 or SEQ ID NO:78. Markers for fertility restoration can also be found on an extended region around these sequences, by extended regions it is intended 5kb around these sequences. Examples of marker on the extended regions are RFL29_S2 or RFL29_S4.

The characterization of the genomic region containing Rf4 *Aegilops speltoides* genetic determinants is detailed in Example section below.

As used herein, the Rf4s locus is located between 6 cM and 43 cM from the chromosomal position delimited by the SNP markers TaContig158085_61_BS00011513 of SEQ ID NO:46 and cfn0864865 of SEQ ID NO:47.

Preferably, the Rf4s locus is located between 6 cM and 36 cM from the chromosomal position delimited by the SNP markers EXCALIBUR_C96134_152 of SEQ ID NO:48 and cfn3133296 of SEQ ID NO:49.

In specific embodiment, the wheat plant restorer comprises any *Ae. Speltoides* SNP on the short arm of the chromosome 6B on the area ranging from 0 to 32 334 597 bases according to IWGSC V1 reference, preferably from the area ranging from 0 to 29 782 272 bases according to IWGSC V1 reference.

In specific embodiment, the wheat plant restorer comprises any *Ae. Speltoides* SNP on the short arm of the chromosome 6B and within the chromosomal interval between 0 to 35.77 cM.

In specific embodiment, the wheat plant restorer of fertility includes one Rf4s restorer allele at the Rf4 *Ae. Speltoides* locus characterized by the presence of one or more of the SNP allele(s) as identified by Table 3.

**Table 3: SNP markers of Rf4s locus**

| SNP# | Marker Name | Marker SEQ ID | Restorer Allele |
|---|---|---|---|
| SNP46 | TaContig158085_61_BS00011513 | 46 | T |
| SNP47 | cfn0864865 | 47 | G |
| SNP48 | EXCALIBUR_C96134_152 | 48 | C |
| SNP49 | cfn3133296 | 49 | G |
| SNP50 | LWE1_chr6B_485210_Rf4S | 50 | T |
| SNP51 | LWE1_chr6B_11287944_Rf4S | 51 | G |
| SNP52 | LWE1_chr6B_19775886_Rf4S | 52 | G |
| SNP53 | LWE1_chr6B_28157776_Rf4S | 53 | C |

Preferably, the wheat plant restorer of fertility according to the present disclosure includes one Rf4s restorer allele at the Rf4 locus characterized by the presence of the SNP53 restorer allele as described in Table 3. More preferably, the wheat plant restorer of fertility is characterized by the haplotype of the SNP53 restorer allele "C".

As used herein, the Rf7 locus is located at most 10 cM from marker cfn0919993 of SEQ ID NO:55. In specific embodiment, the wheat plant restorer of fertility includes one Rf7 restorer allele at the Rf7 locus characterized by the presence of one or more of the SNP allele(s) as identified by Table 4:

**Table 4: SNP markers of Rf7 locus**

| SNP# | Marker Name | Marker SEQ ID | Restorer Allele |
|---|---|---|---|
| SNP54 | cfn0917304 | 54 | T |
| SNP55 | cfn0919993 | 55 | G |
| SNP56 | cfn0920459 | 56 | C |
| SNP57 | cfn0915987 | 57 | G |
| SNP58 | cfn0920253 | 58 | A |
| SNP59 | cfn0448874 | 59 | T |
| SNP60 | cfn0923814 | 60 | C |
| SNP61 | cfn0924180 | 61 | G |
| SNP62 | cfn0919484 | 62 | G |
| SNP64 | LWE1_chr7B_658281643_Rf7 | 263 | G |
| SNP65 | LWE1_chr7B_711539100_Rf7 | 264 | A |

Preferably, the wheat plant restorer of fertility according to the present disclosure includes one Rf7 restorer allele at the Rf7 locus characterized by the presence of one or more SNP restorer allele(s) chosen among SNP54-62 and SNP64-65 of "restorer allele" haplotype, as described in Table 4.

More preferably, the wheat plant restorer of fertility according to the present disclosure includes one Rf7 restorer allele at the Rf7 locus characterized by the presence of the SNP64 restorer allele and/or the SNP65 restorer allele as described in Table 4. More preferably, the wheat plant restorer of fertility is characterized by the haplotype of the SNP64 and SNP65 restorer alleles "G" and "A" respectively.

In specific embodiments, the wheat plant restorer of fertility with Rf7 restorer allele comprises a Rf7 nucleic acid.

As used herein, the term "Rf7 nucleic acid" refers to a nucleic acid comprising a gene encoding a Rf7 protein restorer of fertility of *T. timopheevii* CMS cytoplasm, wherein the corresponding amino acid sequence has at least 95% identity, preferably, 96%, 97%, 98%, 99% or 100% identity to an amino acid sequence selected from the group consisting of SEQ ID NO:79, SEQ ID NO:80 and SEQ ID NO:81.

As used herein, the 6R locus corresponds to the rye introgression T4BS·4BL-6RL from TA5031 line as detailed in Example section. Particularly, the 6R locus is located on chromosome 6R and within the chromosomal interval between 48.9 cM to 114.8 cM (end of the chromosome).

In specific embodiment, the wheat plant restorer of fertility includes the 6R restorer allele at the 6R locus characterized by the presence of the SNP allele as identified by Table 5:

**Table 5: SNP marker of 6R locus**

| SNP# | Marker Name | Marker SEQ ID | Restorer Allele |
|---|---|---|---|
| SNP63 | RFL46_S2 | 63 | A |

In a particular embodiment, the wheat plant restorer of fertility of *T. timopheevii* CMS cytoplasm comprises Rf1, Rf3, Rf4s restorer allele.

In particular, it is hereby included a wheat plant comprising Rf1, Rf3 and Rf4s restorer alleles as provided by the seed samples as deposited on March 22, 2021, under deposit number NCIMB 43746 at the NCIMB collection.

In another particular embodiment, the wheat plant restorer of fertility of *T. timopheevii* CMS cytoplasm comprises Rf1, Rf3, and 6R restorer allele. In particular, it is hereby included a wheat plant comprising Rf1, Rf3 and 6R restorer alleles as provided by the seed samples as deposited on March 22, 2021, under deposit number NCIMB 43747 at the NCIMB collection.

The disclosure also relates to hybrid wheat plants which can be produced by crossing a wheat plant restorer of fertility according to the present disclosure as described above with a second plant.

In certain embodiments, the wheat plant according to the disclosure is alloplasmic and comprises the *T. timopheevii* cytoplasm.

For example, a hybrid wheat plant may be obtained by crossing a wheat plant restorer of fertility according to the present disclosure as described above, for example comprising Rf1, Rf3 and Rf4s restorer alleles or Rf1, Rf3 and 6R restorer alleles, and a wheat plant which does not have said fertility restorer alleles.

It is also disclosed herein a method for producing a wheat hybrid plant comprising the steps of:
a. crossing a sterile female wheat plant comprising the *T.timopheevii* cytoplasm with a fertile male wheat plant of the present disclosure as described above;
b. collecting the hybrid seed;
c. optionally detecting the presence of *T.timopheevii* cytoplasm, and/or the fertility restorer alleles A, B and C as defined above in the hybrid seed;
d. optionally detecting hybridity level of the hybrid seed.

In specific embodiment, step c) comprises the detection of Rf1, Rf3, and Rf4s restorer alleles. According to this embodiment, step c) may optionally further comprise the detection of Rf7 or 6R restorer alleles.

In specific embodiment, step c) comprises the detection of Rf1, Rf3 and 6R restorer alleles.

Therefore, it is also disclosed herein the wheat plants or lines according to the present disclosure developed to obtain such hybrid plants. Such plants or lines typically comprise the cytoplasmic elements necessary for the implementation of the corresponding hybrid system. Preferably, the plants or lines comprise the fertility restorer alleles Rf1, Rf3, Rf4s and T. *timopheevii* cytoplasm, or Rf1, Rf3, 6R and T. *timopheevii* cytoplasm, or Rf3, Rf7, Rf4s and T. *timopheevii* cytoplasm, or also Rf3, Rf7, 6R and T. *timopheevii* cytoplasm.

Alternatively, the detection of the presence of *T. timopheevii* cytoplasm and the restorer alleles (step "c" of the method described above) can be performed on the parent lines in order to check their genotype before to start the cross (step "a") or at every step of their increase.

The T-CMS cytoplasm can be detected either phenotypically wherein a plant bearing rf genes and a T-CMS cytoplasm will be sterile or by molecular means able to detect the *orf256* gene as described in Rathburn and Hedgcoth, 1991 and Song and Hedgcoth, 1994.

### Method of producing and selecting a wheat plant of the disclosure

The present disclosure also relates to the methods to produce the wheat plant with the fertility restorer alleles as described in the previous section.

In one embodiment, the method for producing the wheat plant restorer of fertility comprises the following steps:
a. providing a first wheat plant comprising one or two restorer allele selected among fertility restorer alleles A, B and C as defined above,
b. crossing said first wheat plant with a second wheat plant comprising one or two restorer alleles selected among fertility restorer alleles A, B and C, wherein A, B and C restorer alleles are represented at least once in the panel of restorer alleles provided by the first plant and the second plant,
c. collecting the F1 hybrid seed,
d. obtaining homozygous plants from the F1 plants, and
e. detecting the presence of the fertility restorer alleles A, B and C in the hybrid seed and/or at each generation.

The fertility restorer alleles A, B and C are as previously defined.

In specific embodiments, the fertility restorer allele A is Rf3, the fertility restorer allele B is Rf1, and the fertility restorer allele C is Rf4s.

In specific embodiments, the fertility restorer allele A is Rf3, the fertility restorer allele B is Rf1, and the fertility restorer allele C is 6R.

In specific embodiments, the fertility restorer allele A is Rf3, the fertility restorer allele B is Rf7, and the fertility restorer allele C is Rf4s.

In specific embodiments, the fertility restorer allele A is Rf3, the fertility restorer allele B is Rf7, and the fertility restorer allele C is 6R.

Preferentially, the female plant in step b) is bearing the T-CMS cytoplasm. In this case, the presence of the restorer alleles is assessed at every generation from step b) to step d) by using the markers on the present disclosure and/or by assessing the fertility level.

According to this embodiment, the method may further comprise the detection of Rf1, Rf3, Rf4s, Rf7 and/or 6R restorer alleles in the hybrid seed and/or at each generation.

In another embodiment, the method for producing the wheat plant restorer of fertility comprises the following steps:
a. providing a first wheat plant comprising at least fertility restorer alleles A, B and C as defined above,
b. crossing said first wheat plant with a second wheat plant,
c. collecting the F1 hybrid seed, and
d. obtaining homozygous plants from the F1 plants.

In a variant of this embodiment, the method may comprise the detection of the presence of the Rf1, Rf3 and Rf4s restorer alleles in the hybrid seed and/or at each generation.

In another variant of this embodiment, the method may comprise the detection of the presence of the Rf1, Rf3 and Rf4s restorer alleles in the hybrid seed and/or at each generation.

In another variant of this embodiment, the method may comprise the detection of the presence of the Rf1, Rf3 and 6R restorer alleles in the hybrid seed and/or at each generation

In another variant of this embodiment, the method may comprise the detection of the presence of the Rf1, Rf7 and Rf4s restorer alleles in the hybrid seed and/or at each generation

In another variant of this embodiment, the method may comprise the detection of the presence of the Rf1, Rf7 and 6R restorer alleles in the hybrid seed and/or at each generation.

In another embodiment, the method for producing the wheat plant restorer of fertility comprises the following steps:
a. crossing a first wheat plant having at least the fertility restorer alleles A, B, and C as defined above with a second wheat plant, thereby obtaining a F1 hybrid plant;
b. backcrossing said F1 hybrid with the second wheat plant;
c. selecting the wheat plant restorer of fertility among the wheat plant obtained in step b) by detecting the presence of at the fertility restorer alleles.

According to this embodiment, the method may optionally comprise a step d) of self-crossing the wheat plant to obtain plants homozygous for the restorer fertility alleles A, B, and C.

According to this embodiment, the method may also further comprise one or more step of backcrossing the selected wheat plant by detecting the presence of the restorer alleles initially present in the wheat plant provided at step a).

In a preferred variant of this embodiment, the second wheat plant which is crossed with the first wheat plant having the fertility restorer alleles A, B and C, is an elite wheat line.

Method to generate homozygous plants are generally well known from skilled person of the art. This could be either by repetitive backcross, by double haploid development or by Single Seeds Descent (SSD) methods.

The applicant has deposited a sample of seeds of the disclosed wheat plant with said Rf1, Rf3 and Rf4s restorer alleles, on March 11, 2021, under the Budapest treaty, at NCIMB collection under the number NCIMB 43746, and with said Rf1, Rf3 and 6R restorer alleles, on March 11, 2021, under the Budapest treaty, at NCIMB collection under the number NCIMB 43747.

### Methods of identifying the wheat plant restorer of fertility of the disclosure

The present disclosure further includes and provides methods of identifying the respective Rf1, Rf3, Rf4s, Rf7 and/or 6R restorer alleles as disclosed in the previous sections, and more generally methods of selecting or breeding wheat plants for the presence or absence of the Rf1, Rf3, Rf4s, Rf7 and/or 6R fertility restorer alleles. Such methods of identifying, selecting or breeding wheat plants comprise obtaining one or more wheat plants and assessing their DNA to determine the presence or absence of the Rf1, Rf3, Rf4s, Rf7 and/or 6R fertility restorer alleles contained in the respective locus.

Such methods may be used, for example, to determine which progeny resulting from a cross have the required combination of fertility restorer alleles and accordingly to guide the preparation of plants having the required combination in combination with the presence or absence of other desirable traits.

Accordingly, plants can be identified or selected by assessing them for the presence of one or more individual SNPs appearing in the above Tables 1 to 5, as well as the SNPs in Table 7, for assessing the presence of restorer alleles Rf1, Rf3, Rf4s, Rf7 and/or 6R.

In a specific embodiment, the wheat plant may be identified or selected by assessing the presence of one or more individual SNPs appearing in the above Tables 1 to 3, as well as the SNPs in Table 7, for assessing the presence of restorer alleles Rf1, Rf3, and Rf4s.

In a specific embodiment, the wheat plant may be identified or selected by assessing the presence of one or more individual SNPs appearing in the above Tables 1, 2 and 5, as well as the SNPs in Table 7, for assessing the presence of restorer alleles Rf1, Rf3, and 6R.

In a specific embodiment, the wheat plant may be identified or selected by assessing the presence of one or more individual SNPs appearing in the above Tables 1, 3 and 4, as well as the SNPs in Table 7, for assessing the presence of restorer alleles Rf1, Rf7, and Rf4s.

In a specific embodiment, the wheat plant may be identified or selected by assessing the presence of one or more individual SNPs appearing in the above Tables 1, 4 and 5, as well as the SNPs in Table 7, for assessing the presence of restorer alleles Rf1, Rf7, and 6R.

In a specific embodiment, the wheat plant may be identified or selected by assessing the presence of one or more individual SNPs appearing in the above Tables 1 to 4, as well as the SNPs in Table 7, for assessing the presence of restorer alleles Rf1, Rf3, Rf4s and Rf7.

In a specific embodiment, the wheat plant may be identified or selected by assessing the presence of one or more individual SNPs appearing in the above Tables 1 to 3 and 5, as well as the SNPs in Table 7, for assessing the presence of restorer alleles Rf1, Rf3, Rf4s and 6R.

In a specific embodiment, the wheat plant may be identified or selected by assessing the presence of one or more individual SNPs appearing in the above Tables 1, 4 and 5, as well as the SNPs in Table 7, for assessing the presence of restorer alleles Rf1, Rf3, Rf7 and 6R.

More generally, it is disclosed herein the specific means for detecting the restorer alleles in a wheat plant, more specifically Rf1, Rf3, Rf4s, Rf7 and 6R restorer alleles and their combinations.

Said means thus include any means suitable for detecting the following SNP markers within one or more of the following markers: SEQ ID NOs 1-65.

Any method known in the art may be used in the art to assess the presence or absence of a SNP. Some suitable methods include, but are not limited to, sequencing, hybridization assays, polymerase chain reaction (PCR), ligase chain reaction (LCR), and genotyping-by-sequence (GBS), or combinations thereof.

Different PCR based methods are available to the person skilled of the art. One can use the RT-PCR method or the Kaspar method from KBioscience (LGC Group, Teddington, Middlesex, UK).

The KASP^{™} genotyping system uses three target specific primers: two primers, each of them being specific of each allelic form of the SNP (Single Nucleotide Polymorphism) and one other primer to achieve reverse amplification, which is shared by both allelic form. Each target specific primer also presents a tail sequence that corresponds with one of two FRET probes: one label with FAM^{®} dye and the other with HEX^{®} dye.

Successive PCR reactions are performed. The nature of the emitted fluorescence is used to identify the allelic form or forms present in the mix from the studied DNA.

The primers identified in Table 6 are particularly suitable for use with the KASP^{™} genotyping system. Of course, the skilled person may use variant primers or nucleic acid probes of the primers as identified in Table 6, said variant primers or nucleic acid probes having at least 90%, and preferably 95% sequence identity with any one of the primers as identified in Table 6, or with the DNA genomic fragment amplified by the corresponding set of primers as identified in Table 6.

Percentage of sequence identity as used herein is determined by calculating the number of matched positions in aligned nucleic acid sequences, dividing the number of matched positions by the total number of aligned nucleotides, and multiplying by 100. A matched position refers to a position in which identical nucleotides occur at the same position in aligned nucleic acid sequences. For example, nucleic acid sequences may be aligned using the BLAST 2 sequences (BI2seq) using BLASTN algorithms (www.ncbi.nlm.nih.gov).

As used herein, a primer encompasses any nucleic acid that is capable of priming the synthesis of a nascent nucleic acid in a template-dependent process, such as PCR. Typically, primers are oligonucleotides from 10 to 30 nucleotides, but longer sequences can be employed. Primers may be provided in double-stranded form though single-stranded form is preferred. Alternatively, nucleic acid probe can be used. Nucleic acid probe encompasses any nucleic acid of at least 30 nucleotides and which can specifically hybridizes under standard stringent conditions with a defined nucleic acid. Standard stringent conditions as used herein refers to conditions for hybridization described for example in Sambrook et al 1989 which can comprise 1) immobilizing plant genomic DNA fragments or library DNA on a filter 2) prehybridizing the filter for 1 to 2 hours at 65°C in 6x SSC 5x Denhardt's reagent, 0.5% SDS and 20mg/ml denatured carrier DNA 3) adding the probe (labeled) 4) incubating for 16 to 24 hours 5) washing the filter once for 30min at 68°C in 6x SSC, 0.1% SDS 6) washing the filter three times (two times for 30min in 30ml and once for 10 min in 500ml) at 68°C in 2x SSC 0.1% SDS.

In specific embodiments, said primers for detecting the SNP markers of the present disclosure (specific for each allele "X" or "Y" or common) are as listed in the following Table 6:

**Table 6: Primers for use in detecting fertility restorer SNP markers of the invention (as indicated in the primer name)**

| **SEQ ID NO:** | MARKER NAME | Sequence |
|---|---|---|
| 82 | cfn0523109 Allele X | GAAGGTGACCAAGTTCATGCTGGTGAACAAAACAGGCCT ACAATCA |
| 83 | 276I13_96B22_97797 Allele X | GAAGGTGACCAAGTTCATGCTGTACTATGGCTATGTCTCT GAATGC |
| 84 | cfn0522096 Allele X | GAAGGTGACCAAGTTCATGCTAGTAGAATACCACCCAATA AATCACTG |
| 85 | cfn0527763 Allele X | GAAGGTGACCAAGTTCATGCTATCTAGCCACGCAAATGC CCGT |
| 86 | 104A4_105172 Allele X | GAAGGTGACCAAGTTCATGCTGTCGMACCCAATGAATAA TGTTT |
| 87 | 104A4_105588 Allele X | GAAGGTGACCAAGTTCATGCTGTTCCTTGTGACATGTACT CATAA |
| 88 | cfn0373248 Allele X | GAAGGTGACCAAGTTCATGCTAACAACAATTAYGAGGATC AAATGGTCA |
| 89 | cfn1097828 Allele X | GAAGGTGACCAAGTTCATGCTGGTTCCTGAGAGAGCAAC CA |
| 90 | cfn0527067 Allele X | GAAGGTGACCAAGTTCATGCTCAAATTACTTTTGTTCTTTT ATTTTTTTCGAAT |
| 91 | cfn0528390 Allele X | GAAGGTGACCAAGTTCATGCTAAAAACATCTATTCCAAGC AAGTATTAGTAAT |
| 92 | BWS0267 Allele X | GAAGGTGACCAAGTTCATGCTTCAGCTGCATAAAAMCA GAATACCA |
| 93 | cfn0527718 Allele X | GAAGGTGACCAAGTTCATGCTAATTGTTCACAACATGGAC ATGAGAAC |
| 94 | cfn0524469 Allele X | GAAGGTGACCAAGTTCATGCTTCAGCTGCATAAAAMCA GAATACCA |
| 95 | cfn0524921 Allele X | GAAGGTGACCAAGTTCATGCTATTGTTTCCATGTTAAGCT TATATTGTGCA |
| 96 | cfn1122326 Allele X | GAAGGTGACCAAGTTCATGCTGAATCTGATTAAGACGCT GGAGAAC |
| 97 | RFL79_S7 Allele X | GAAGGTGACCAAGTTCATGCTAAGCTTGATAAGGCTATG CTTATATTTAG |
| 98 | cfn1252000 Allele X | GAAGGTGACCAAGTTCATGCTGTTAATGCTGTAGCCATTC TTGCAA |
| 99 | IWB14060* Allele X | GAAGGTGACCAAGTTCATGCTCATTCGACGCGTCTTCCG CAATA |
| 100 | cfn1249269 Allele X | GAAGGTGACCAAGTTCATGCTGATTCAAAGAGGTGACAA ATATGTGTACT |
| 101 | 219K1_166464 Allele X | GAAGGTGACCAAGTTCATGCTCCTGAGCTGGGCTGCACC |
| 102 | 219K1_158251 Allele X | GAAGGTGACCAAGTTCATGCTCCTGGAGATGGATCCGGT CAG |
| 103 | 219K1_111446 Allele X | GAAGGTGACCAAGTTCATGCTAGAATCGTTCTTCGAGAA GCACTCA |
| 104 | 219K1_110042 Allele X | GAAGGTGACCAAGTTCATGCTACGGAATCGAGTCAACCA ATTCCT |
| 105 | 219K1_11 0005 Allele X | GAAGGTGACCAAGTTCATGCTGCCTTTTCTTCTTCCAGCA TCTAC |
| 106 | 219K1_107461 Allele X | GAAGGTGACCAAGTTCATGCTATATTGTTTGTATTAAAAA GTTGTGTGTTTTGA |
| 107 | 219K1_99688 Allele X | GAAGGTGACCAAGTTCATGCTGTTGCCCTGCGCAAAATC AAACTT |
| 108 | 219K1_37 Allele X | GAAGGTGACCAAGTTCATGCTAAAGGGCTATCCTGGTGA ACAAC |
| 109 | cfn1270524 Allele X | GAAGGTGACCAAGTTCATGCTAAATGCCTAGTCTATACCT GATAAACTAAA |
| 110 | 136H5_3M5_7601 Allele X | GAAGGTGACCAAGTTCATGCTCGTCCCCCATGGCACCTG T |
| 111 | cfn1288811 Allele X | GAAGGTGACCAAGTTCATGCTTAATTTGGTTAACCAAATC CTTTTTGATTTTT |
| 112 | 136H5_3M5_89176 Allele X | GAAGGTGACCAAGTTCATGCTGGATTTTCTCACCGGCAT CTCCA |
| 113 | 136H5_3M5_89263 Allele X | GAAGGTGACCAAGTTCATGCTTCCCATGTTCTTTTTTTGC TCAAAAC |
| 114 | 136H5_3M5_138211 Allele X | GAAGGTGACCAAGTTCATGCTACTGGGTGCAAAGCCAAG ATGATT |
| 115 | cfn0556874 Allele X | GAAGGTGACCAAGTTCATGCTAAAGAGCATGTCAGACAC AATGCAG |
| 116 | 136H5_3M5_64154 Allele X | GAAGGTGACCAAGTTCATGCTGGCGAAACTTCGCCGCGA TAAAT |
| 117 | 136H5_3M5_68807 Allele X | GAAGGTGACCAAGTTCATGCTCAAGTTGCTCTTAATTATC TGTGCGTA |
| 118 | 136H5_3M5_77916 Allele X | GAAGGTGACCAAGTTCATGCTATAGCAAGTAGAGTTAACT TATCAAGTTATTA |
| 119 | cfn1246088 Allele X | GAAGGTGACCAAGTTCATGCTGACATCTGATGAGCCAGC ATACA |
| 120 | cfn1287194 Allele X | GAAGGTGACCAAGTTCATGCTACCTCCTCCGTATCTGAT GGC |
| 121 | cfn1258380 Allele X | GAAGGTGACCAAGTTCATGCTATCTACTCATCTATTGCAG ATGCTCTT |
| 122 | IWB72107* Allele X | GAAGGTGACCAAGTTCATGCTGATGACATGGAGGATTAT ATCGACGA |
| 123 | BS00090770 Allele X | GAAGGTGACCAAGTTCATGCTGGTCGTAGCACATAGCCG TTTAC |
| 124 | cfn1239345 Allele X | GAAGGTGACCAAGTTCATGCTGGCTTCTTTTTCTCCCTA TAATATGGA |
| 125 | RFL29_S2 Allele X | GAAGGTGACCAAGTTCATGCTCGGGCAACTCTCTTCTTCT TAATCAA |
| 126 | RFL29_S4 Allele X | GAAGGTGACCAAGTTCATGCTATGATGACTCCATGAGGG TGGC |
| 127 | cfn0917304 Allele X | GAAGGTGACCAAGTTCATGCTGTGGTGGCGCTCTACCCG |
| 128 | cfn0919993 Allele X | GAAGGTGACCAAGTTCATGCTAAGTCATCGACTTACATGC TTCTTTG |
| 129 | cfn0920459 Allele X | GAAGGTGACCAAGTTCATGCTAGCCAAGGAAGCCCAGAT TTTC |
| 130 | cfn0915987 Allele X | GAAGGTGACCAAGTTCATGCTAGATCATTACCCAACGGC CAATG |
| 131 | cfn0920253 Allele X | GAAGGTGACCAAGTTCATGCTGGTCATCCAAACATTTACA TCGTTA |
| 132 | cfn0448874 Allele X | GAAGGTGACCAAGTTCATGCTCTTTGTTTCTAAATAGCTG CGGCC |
| 133 | cfn0923814 Allele X | GAAGGTGACCAAGTTCATGCTCCAAGTCGCAAATGTAAG GTCAGA |
| 134 | cfn0924180 Allele X | GAAGGTGACCAAGTTCATGCTTCCTCTTTTCATCATGCAC CATTA |
| 135 | cfn0919484 Allele X | GAAGGTGACCAAGTTCATGCTAAATGCAAGTGGCGAATC TTATCTCTA |
| 136 | Excalibur_c96134_152 Allele X | GAAGGTGACCAAGTTCATGCTACTCTGGTGACACCATGT AACTTC |
| 137 | cfn3133296 Allele X | GAAGGTGACCAAGTTCATGCTCCAAGTGTCCCTCCTTGA GTCA |
| 138 | LWE1_chr6B_485210_Rf4S Allele X | GAAGGTGACCAAGTTCATGCTCATACTTGTAGAGATCGTC ACCC |
| 139 | LWE1_chr6B_11287944_Rf4S Allele X | GAAGGTGACCAAGTTCATGCTCTTCTGTTTAGGACTACAC ATCAACT |
| 140 | LWE1_chr6B_19775886_Rf4S Allele X | GAAGGTGACCAAGTTCATGCTAAGGGCGCCGGCACTGG T |
| 141 | LWE1_chr6B_28157776_Rf4S Allele X | GAAGGTGACCAAGTTCATGCTTTAGAAACGATCTGCTTAC TGATTACTAT |
| 142 | RFL46_S2 Allele X | GAAGGTGACCAAGTTCATGCTGGCCAGAGCTATGGACAA AGCAA |
| 143 | cfn0523109 Allele Y | GAAGGTCGGAGTCAACGGATTGTGAACAAAACAGGCCTA CAATCC |
| 144 | 276I13_96B22_97797 Allele Y | GAAGGTCGGAGTCAACGGATTAAGTACTATGGCTATGTC TCTGAATGT |
| 145 | cfn0522096 Allele Y | GAAGGTCGGAGTCAACGGATTAGTAGAATACCACCCAAT AAATCACTC |
| 146 | cfn0527763 Allele Y | GAAGGTCGGAGTCAACGGATTCTAGCCACGCAAATGCCC GC |
| 147 | 104A4_105172 Allele Y | GAAGGTCGGAGTCAACGGATTCTGTCGMACCCAATGAAT AATGTTC |
| 148 | 104A4_105588 Allele Y | GAAGGTCGGAGTCAACGGATTGTTCCTTGTGACATGTAC TCATAC |
| 149 | cfn0373248 Allele Y | GAAGGTCGGAGTCAACGGATTAACAACAATTAYGAGGAT CAAATGGTCT |
| 150 | cfn1097828 Allele Y | GAAGGTCGGAGTCAACGGATTGGTTCCTGAGAGAGCAAC CG |
| 151 | cfn0527067 Allele Y | GAAGGTCGGAGTCAACGGATTCAAATTACTTTTGTTCTTT TATTTTTTTCGAAC |
| 152 | cfn0528390 Allele Y | GAAGGTCGGAGTCAACGGATTAAACATCTATTCCAAGCA AGTATTAGTAAC |
| 153 | BWS0267 Allele Y | GAAGGTCGGAGTCAACGGATTCAGCTGCATAAAAMCAG AATACCG |
| 154 | cfn0527718 Allele Y | GAAGGTCGGAGTCAACGGATTATAAATTGTTCACAACATG GACATGAGAAT |
| 155 | cfn0524469 Allele Y | GAAGGTCGGAGTCAACGGATTGCACGTAGTAAGTATTGA TTTTTCTGTT |
| 156 | cfn0524921 Allele Y | GAAGGTCGGAGTCAACGGATTGTTTCCATGTTAAGCTTAT ATTGTGCG |
| 157 | cfn1122326 Allele Y | GAAGGTCGGAGTCAACGGATTGGAATCTGATTAAGACGC TGGAGAAT |
| 158 | RFL79_S7 Allele Y | GAAGGTCGGAGTCAACGGATTGAAGCTTGATAAGGCTAT GCTTATATTTAA |
| 159 | cfn1252000 Allele Y | GAAGGTCGGAGTCAACGGATTGTTAATGCTGTAGCCATT CTTGCAG |
| 160 | IWB14060* Allele Y | GAAGGTCGGAGTCAACGGATTCGACGCGTCTTCCGCAAT G |
| 161 | cfn1249269 Allele Y | GAAGGTCGGAGTCAACGGATTCAAAGAGGTGACAAATAT GTGTACC |
| 162 | 219K1_166464 Allele Y | GAAGGTCGGAGTCAACGGATTGCCTGAGCTGGGCTGCA CT |
| 163 | 219K1_158251 Allele Y | GAAGGTCGGAGTCAACGGATTCCTGGAGATGGATCCGGT CAA |
| 164 | 219K1_111446 Allele Y | GAAGGTCGGAGTCAACGGATTAATCGTTCTTCGAGAAGC ACTCC |
| 165 | 219K1_11 0042 Allele Y | GAAGGTCGGAGTCAACGGATTCGGAATCGAGTCAACCAA TTCCC |
| 166 | 219K1_11 0005 Allele Y | GAAGGTCGGAGTCAACGGATTCGCCTTTTCTTCTTCCAG CATCTAT |
| 167 | 219K1_107461 Allele Y | GAAGGTCGGAGTCAACGGATTATATTGTTTGTATTAAAAA GTTGTGTGTTTTGC |
| 168 | 219K1_99688 Allele Y | GAAGGTCGGAGTCAACGGATTGCCCTGCGCAAAATCAAA CTC |
| 169 | 219K1_37 Allele Y | GAAGGTCGGAGTCAACGGATTACAAAGGGCTATCCTGGT GAACAAT |
| 170 | cfn1270524 Allele Y | GAAGGTCGGAGTCAACGGATTAAATGCCTAGTCTATACC TGATAAACTAAT |
| 171 | 136H5_3M5_7601 Allele Y | GAAGGTCGGAGTCAACGGATTGTCCCCCATGGCACCTGC |
| 172 | cfn1288811 Allele Y | GAAGGTCGGAGTCAACGGATTAATTTGGTTAACCAAATCC TTTTTGATTTTG |
| 173 | 136H5_3M5_89176 Allele Y | GAAGGTCGGAGTCAACGGATTTTCTCACCGGCATCTCCG |
| 174 | 136H5_3M5_89263 Allele Y | GAAGGTCGGAGTCAACGGATTCTTCCCATGTTCTTTTTTT GCTCAAAAT |
| 175 | 136H5_3M5_138211 Allele Y | GAAGGTCGGAGTCAACGGATTACTGGGTGCAAAGCCAAG ATGATA |
| 176 | cfn0556874 Allele Y | GAAGGTCGGAGTCAACGGATTGAAAGAGCATGTCAGACA CAATGCAA |
| 177 | 136H5_3M5_64154 Allele Y | GAAGGTCGGAGTCAACGGATTGCGAAACTTCGCCGCGAT AAAC |
| 178 | 136H5_3M5_68807 Allele Y | GAAGGTCGGAGTCAACGGATTAAGTTGCTCTTAATTATCT GTGCGTG |
| 179 | 136H5_3M5_77916 Allele Y | GAAGGTCGGAGTCAACGGATTAGCAAGTAGAGTTAACTT ATCAAGTTATTG |
| 180 | cfn1246088 Allele Y | GAAGGTCGGAGTCAACGGATTGACATCTGATGAGCCAGC ATACC |
| 181 | cfn1287194 Allele Y | GAAGGTCGGAGTCAACGGATTCACCTCCTCCGTATCTGA TGGT |
| 182 | cfn1258380 Allele Y | GAAGGTCGGAGTCAACGGATTCTACTCATCTATTGCAGAT GCTCTG |
| 183 | IWB72107* Allele Y | GAAGGTCGGAGTCAACGGATTATGACATGGAGGATTATA TCGACGG |
| 184 | BS00090770 Allele Y | GAAGGTCGGAGTCAACGGATTAGGTCGTAGCACATAGCC GTTTAT |
| 185 | cfn1239345 Allele Y | GAAGGTCGGAGTCAACGGATTGCTTCTTTTTCTCCCTAT AATATGGG |
| 186 | RFL29_S2 Allele Y | GAAGGTCGGAGTCAACGGATTGGGCAACTCTCTTCTTCT TAATCAG |
| 187 | RFL29_S4 Allele Y | GAAGGTCGGAGTCAACGGATTAATGATGACTCCATGAGG GTGGT |
| 188 | cfn0920459 Allele Y | GAAGGTCGGAGTCAACGGATTAGCCAAGGAAGCCCAGAT TTTG |
| 189 | cfn0915987 Allele Y | GAAGGTCGGAGTCAACGGATTCAGATCATTACCCAACGG CCAATT |
| 190 | cfn0920253 Allele Y | GAAGGTCGGAGTCAACGGATTGGTCATCCAAACATTTAC ATCGTTC |
| 191 | cfn0448874 Allele Y | GAAGGTCGGAGTCAACGGATTCTCTTTGTTTCTAAATAGC TGCGGCT |
| 192 | cfn0923814 Allele Y | GAAGGTCGGAGTCAACGGATTCAAGTCGCAAATGTAAGG TCAGC |
| 193 | cfn0924180 Allele Y | GAAGGTCGGAGTCAACGGATTCTTCCTCTTTTCATCATGC ACCATTG |
| 194 | cfn0919484 Allele Y | GAAGGTCGGAGTCAACGGATTATGCAAGTGGCGAATCTT ATCTCTG |
| 195 | Excalibur_c96134_152 Allele Y | GAAGGTCGGAGTCAACGGATTGACTCTGGTGACACCATG TAACTTT |
| 196 | cfn3133296 Allele Y | GAAGGTCGGAGTCAACGGATTCAAGTGTCCCTCCTTGAG TCG |
| 197 | LWE1_chr6B_485210_Rf4S Allele Y | GAAGGTCGGAGTCAACGGATTGTCATACTTGTAGAGATC GTCACCA |
| 198 | LWE1_chr6B_11287944_Rf4S Allele Y | GAAGGTCGGAGTCAACGGATTCTTCTGTTTAGGACTACA CATCAACC |
| 199 | LWE1_chr6B_19775886_Rf4S Allele Y | GAAGGTCGGAGTCAACGGATTAGGGCGCCGGCACTGGC |
| 200 | LWE1_chr6B_28157776_Rf4S Allele Y | GAAGGTCGGAGTCAACGGATTAGAAACGATCTGCTTACT GATTACTAG |
| 201 | RFL46_S2 Allele Y | GAAGGTCGGAGTCAACGGATTGCCAGAGCTATGGACAAA GCAG |
| 202 | cfn0523109 Common | GTGTGTGCTAATGTGGATATACGTAAGTT |
| 203 | 276I13_96B22_97797 Common | ACGACAATATAGACAAATAAAACCAAACAA |
| 204 | cfn0522096 Common | AAGTAGTACTCGTAGAGAGTTAACACAGA |
| 205 | cfn0527763 Common | CCTTGTCCACCGAGACATGTACAAA |
| 206 | 104A4_105172 Common | GCCATCCTCTCGGAGCCAGAA |
| 207 | 104A4_105588 Common | CAAGGATGGGGAGTATATGGCTCTT |
| 208 | cfn0373248 Common | ATCATTGCCACGRAAAAAATCTCACAAGAT |
| 209 | cfn1097828 Common | GCTTCCTCTCGGTAGCGATGGAT |
| 210 | cfn0527067 Common | ATATGATTCACCCTAGATCCTTCACCTTA |
| 211 | cfn0528390 Common | AATAACTCTTGTACTTCAGGATGAACGTTT |
| 212 | BWS0267 Common | CTGCGTTAAGGTTCAGGCAACTGAT |
| 213 | cfn0527718 Common | GTTTCCTCCAATGTTCTTCCC |
| 214 | cfn0524469 Common | GCCAATTTTCAAATCTAAGTCCACAGAGA |
| 215 | cfn0524921 Common | GCCCTTTGGTAATTCCATTTCAATCTTTT |
| 216 | cfn1122326 Common | CAGATGGCCTAGTCGTGACATATCTT |
| 217 | RFL79_S7 Allele Common | CTCACTCCTTGTTTCTGCATATCT |
| 218 | cfn1252000 Common | GTGCCCATAAGACGACTGGGACAA |
| 219 | IWB14060* Common | CCGCGGCCGAAGCAGGCAA |
| 220 | cfn1249269 Common | TAAAAGAACACAAATGTGGCCCTAGTGAT |
| 221 | 219K1_166464 Common | GACCGTGGTATATGCCACCACGTT |
| 222 | 219K1_158251 Common | TCCTCACAAATCACGGGCCCCT |
| 223 | 219K1_111446 Common | AATATGATACAGACCCAAGACAAACCATTT |
| 224 | 219K1_110042 Common | G CATCTTCAAGG GAG CCACTCAAAA |
| 225 | 219K1_110005 Common | TTGACTCGATTCCGTGTGAGGCTAA |
| 226 | 219K1_107461 Common | GTTGATGCGAATTTGAAAATGACATAATAA |
| 227 | 219K1_99688 Common | GGGCGGGACCTGACTTGATGAT |
| 228 | 219K1_37 Common | GGCTTCATTATCAAATTCTGACCCATCTT |
| 229 | cfn1270524 Common | TGTACCGAAACTCAACCAAATGACCATTT |
| 230 | 136H5_3M5_7601 Common | CTTCTCTGTGGCCGAAAACCTCTT |
| 231 | cfn1288811 Common | GCACAATGTTTGACATTCGGTTTTCTAGTT |
| 232 | 136H5_3M5_89176 Common | CCTACCATCCTTAAATACTCTTGCTCAAA |
| 233 | 136H5_3M5_89263 Common | AAGCAACTAGAAAAATATTTGGACTAGCAT |
| 234 | 136H5_3M5_138211 Common | CCTCCCAACGGCCATCAATCAATTT |
| 235 | cfn0556874 Common | CCTGCTGGAAATGGGATTTCTTGTTTATT |
| 236 | 136H5_3M5_64154 Common | GATCATCGGGGAACCTGATGATAGTT |
| 237 | 136H5_3M5_68807 Common | TTGGTTGGTTACGTCAGGTTAAGACTTA |
| 238 | 136H5_3M5_77916 Common | GCTKTAGACTCTAAGTACCACAGAAGAA |
| 239 | cfn1246088 Common | GGGACGTGGAATTTGGAAAGACACAT |
| 240 | cfn1287194 Common | CAGAAGGCACTGGGAGGGGATT |
| 241 | cfn1258380 Common | TATAGGAGTGATAGCACCACACAATTCAT |
| 242 | IWB72107* Common | ATACATGTCGGCGTCCCAGTCC |
| 243 | BS00090770 Common | GAAACATTCCTTCGGACAACTATGCATTA |
| 244 | cfn1239345 Common | ACCCTCGCTGCAGTTCCTTCTTAAA |
| 245 | RFL29_S2 Allele Common | TTTAGGACCTCCAGTGCATTTAACTCTTT |
| 246 | RFL29_S4 Allele Common | CAGTGCAACCTGCGGAGAGCAT |
| 247 | cfn0917304 Common | CAACTGCTTGGAGAAAGGCAACACAA |
| 248 | cfn0919993 Common | CCATTAACAAGTACTGCATAGGTGCATAT |
| 249 | cfn0920459 Common | CCTCCTCCT AATTAAGCTCCTATAGAT A |
| 250 | cfn0915987 Common | AAACGTGCAACGAGGCAAACCTCAT |
| 251 | cfn0920253 Common | GCCGCATGGTTTGGGCGGAAA |
| 252 | cfn0448874 Common | GTGCCTCTAGGTTCAACATAAATTTAGGTA |
| 253 | cfn0923814 Common | GATTTTCATTATCATGATCATCATTCATTT |
| 254 | cfn0924180 Common | AATGGCTTCAGACAAAATAAGAGGGAGAT |
| 255 | cfn0919484 Common | TCTCGCCTTTGTTTTGCCAAATGGTATAA |
| 256 | Excalibur_c96134_152 Common | CAAACTCCAACGGGTGGTGCGT |
| 257 | cfn3133296 Common | GCAATCCACCACTGTGGTACAACTT |
| 258 | LWE1_chr6B_485210_Rf4S Common | GGCACGATGACAGTAATGGGATGTT |
| 259 | LWE1_chr6B_11287944_Rf4S Common | AGAGTACACAGCATTTTCCCAGGAATATA |
| 260 | LWE1_chr6B_19775886_Rf4S Common | GTGGCAAGCAGATCATGACAGGTT |
| 261 | LWE1_chr6B_28157776_Rf4S Common | CTTGACGCATAAGGTGAAAGCCTGAA |
| 262 | RFL46_S2 Common | CCTTTATCAATCATCTGCCGGAGGAA |
| 265 | LWE1_chr7B_658281643_Rf7 Allele X | GAAGGTGACCAAGTTCATGCTGGGGTCTGTAAACTTGTG ACGGA |
| 266 | LWE1_chr7B_658281643_Rf7 Allele Y | GAAGGTCGGAGTCAACGGATTGGGTCTGTAAACTTGTGA CGGC |
| 267 | LWE1_chr7B_658281643_Rf7 Common | CTCTAACGATTCTTACACACGCACCAA |
| 268 | LWE1_chr7B_711539100_Rf7 Allele X | GAAGGTGACCAAGTTCATGCTCCTCTTCTCCAGATAATCA ATCCTC |
| 269 | LWE1_chr7B_711539100_Rf7 Allele Y | GAAGGTCGGAGTCAACGGATTCCTCTTCTCCAGATAATC AATCCTA |
| 270 | LWE1_chr7B_711539100_Rf7 Common | GGCGACGGAGCTCGATGAGAAA |

### Methods of Use of the wheat plants of the disclosure

The plant according to the disclosure can be crossed, with any another inbred line, in order to produce a new line comprising either an increase or a decrease in the fertility level.

Alternatively, a genetic trait which has been engineered into a particular line using the foregoing techniques could be moved into another line using traditional backcrossing techniques that are well known in the plant breeding arts. For example, a backcrossing approach could be used to move an engineered trait from a public, non-elite inbred line into an elite inbred line, or from an inbred line containing a foreign gene in its genome into an inbred line or lines which do not contain that gene.

The wheat plant of the disclosure is also a wheat plant wherein one or more desired traits have further been introduced through backcrossing methods, whether such trait is a naturally occurring one or not.

The disclosure also relates to the use of the wheat plant as described above or its seeds, for food applications, preferably for flour production and for feed applications, or for breeding applications, for example in a method for improving agronomical value of a wheat plant, line, hybrid or variety.

As used herein, breeding applications encompass pedigree breeding to improve the agronomical value of a plant, line, hybrid, or variety.

Seeds harvested from plants described herein can be used to make flour by any available techniques in the art. The wheat plants or their flour are also useful as food product.

### SEQUENCES OF SNP MARKERS

**Table 7: Sequences of SNP markers**

| **Marker ID** | **Allele X** | **Allele Y** | **Sequence** |
|---|---|---|---|
| 276I13_96B22_977 97 | C | T | AAATTCGACAAGTACTATGGCTATGTCTCTGAAT G[C/T]TTGTTTGGTTTTATTTGTCTATATTGTCGTT GTAT |
| cfn0522096 | C | G | ATGCAAAGTAGTACTCGTAGAGAGTTAACACAGA C[C/G]AGTGATTTATTGGGTGGTATTCTACTTGAT ATTTG |
| cfn0527763 | T | C | ATAAAGAAAAGTAGAGGAAGCTTATGAATAAAAT GGAAAAGGAATTCAAAATTGCCGATAAATATAAAA CTCATAACAAATCTAGCCACGCAAATGCCCG[T/C] GCCGCTCTGCTCGTTTGTACATGTCTCGGTGGAC AAGGAAGAACCCAACAATTGCACAGGTCAATCTT ATCCAGCAAAACAAGGAAGCAAACCAAACAGG |
| 104A4_105172 | TG | CA | ATGTTGCCTCTCGCTAGCCGCTGTCGMACCCAAT GAATAATGTT[TG/CA]TGGGTTCTGGCTCCGAGAG GATGGCCGGCTYCCC |
| 104A4_105588 | A | C | GTTCCTTGTGACATGTACTCATA[A/C]ACAAGAGC CATATACTCCCCATCCTTGCA |
| cfn0373248 | T | A | GACATAATGTGTAATAACAGCCCATAATGCAATAA ATATCAATATAAAAGCATGATGCAAAATGGACGTA TCATTGCCACGRAAAAAATCTCACAAGATG[T/A]G ACCATTTGATCCTCRTAATTGTTGTTCTAGACCCA CTCCTAAGTMTAACATTCTTTATGTCTATYCTTCA AATCCCGAAGAGTAATGAAAACTATCGAA |
| cfn1097828 | T | C | CCATGAGTACCCGCTACTATCGATCTCCCTCCTC CCTGTAGGAGGCCTACGAACGATGCCCTCAGGT CCTGCTTCCTCTCGGTAGCGATGGATCCACCTG[ T/C]GGTTGCTCTCTCAGGAACCAGTGTTGGCGGC GGCTCATCCGGGGCGCTGGATCTTGGTGATGTG CTGGAACAACTCAACTTGGAAGACGAAGAATTTG AT |
| cfn0527067 | A | G | GACAATATGATTCACCCTAGATCCTTCACCTTACA [A/G]TTCGAAAAAAATAAAAGAACAAAAGTAATTTG ACA |
| cfn0528390 | A | G | ACGAAGATGAGGAAGGTCTTCATGTTGGGTTTAT G[A/G]TTACTAATACTTGCTTGGAATAGATGTTTTT GATC |
| BWS0267 | A | G | GTTACCCCAATATGCTCCCTCCTTGCACATTTTCT TCAGCTGCATAAAAMCAGAATACC[A/G]CATCAG TTGCCTGAACCTTAACGCAGGTGCAGAAATAAGG CGACATAATTTYCACTAATC |
| cfn0527718 | C | T | AGGAAAATAAATTGTTCACAACATGGACATGAGA A[C/T]GGGGCAACCAAAAAGGAAGAACATTGGA GGAAAC |
| cfn0524469 | G | T | TTTGTACTGCACGTAGTAAGTATTGATTTTTCTGT[ G/T]TGCTCTCTGTGGACTTAGATTTGAAAATTGGC CTT |
| cfn0524921 | A | G | ATGCACATTGTTTCCATGTTAAGCTTATATTGTGC[ A/G]TAACTCAAAAGATTGAAATGGAATTACCAAAG GGC |
| cfn1122326 | C | T | ACTGACTGTTGGAATCTGATTAAGACGCTGGAGA A[C/T]CCGAGCCAAGATATGTCACGACTAGGCCA TCTGGA |
| RFL79_S7 | G | A | TGGGAAGCTTGATAAGGCTATGCTTATATTTA[G/A ]AGATATGCAGAAACAAGGAGTGAG |
| cfn1252000 | A | G | AATCAGATCCTGTTAATGCTGTAGCCATTCTTGCA [A/G]GCGACACCTTGTCCCAGTCGTCTTATGGGC ACTTA |
| IWB14060 | A | G | GGCAGAGCCGGTCGACGGAGAGGAGCGCCATT CGACGCGTCTTCCGCAAT[A/G]TGTTTGCCTGCTT CGGCCGCGGCCATTCGGCGAGCTCCCACGCTTC GTCC |
| cfn1249269 | A | G | CGTTTAAAAGAACACAAATGTGGCCCTAGTGATC A[A/G]GTACACATATTTGTCACCTCTTTGAATCTTA CTTA |
| 219K1_166464 | C | T | CGGGCTGATGAGGCTCTCGACGTGCTGCTTCAC AGGATGCCTGAGCTGGGCTGCAC[C/T]CCCAACG TGGTGGCATATACCACGGTCATCCACGGCTTCTT TAAGGAAGGC |
| 219K1_158251 | G | A | GCGCTATCCGGCGTCGTGTTCCCTCTTGGGGGA ATCGTCCTGGAGATGGATCCGGTCA[G/A]AGGGG CCCGTGATTTGTGAGGATGTGTGTGTTGTTTCCC GAAAGGCG |
| 219K1_111446 | A | C | CTTTGACCTTAAATTCTTGTACTAATTTAGCAGAA TCGTTCTTCGAGAAGCACTC[A/C]AAAAATGGTTT GTCTTGGGTCTGTATCATATTTTCTCTGAACAAAC AGGCGTGA |
| 219K1_110042 | T | C | GACTTAGCCTCACACGGAATCGAGTCAACCAATT CC[T/C]GTCGGTTTTGAGTGGCTCCCTTGAAGATG CAATCGTTTTCAGCATGGTCAGATTAATCAGCGA GCGTGC |
| 219K1_110005 | C | T | CATGTAGTGGCTGGCGTCTAAGCGCCTTTTCTTC TTCCAGCATCTA[C/T]GACTTAGCCTCACACGGAA TCGAGTCAACCAATTCCTGTCGGTTTTGAGTGGC TCCCTTGAAGATG |
| 219K1_107461 | A | C | GTCGTATATATTGTTTGTATTAAAAAGTTGTGTGT TTTG[A/C]GTCATAATTTTTAAAATATTATTATGTCA TTTTCAAATTCGCATCAAC |
| 219K1_99688 | T | C | AATCTTCTTGACTTCATCCATCCGCCTTGTTGCCC TGCGCAAAATCAAACT[T/C]CCCCGTCCTTATCAT CAAGTCAGGTCCCGCCCTGGGCAGAGAGAG |
| 219K1_37 | C | T | CGGCAGATATCACAAAGGGCTATCCTGGTGAACA A[C/T]AAGATGGGTCAGAATTTGATAATGAAGCCT CAAGCCC |
| cfn1270524 | A | T | AATAGATGCACGCATCGGCGACCATTTTTAGTA CTTTTTGCCTTTTTTGAAAATTTTGTCATTAAAAGA CAAATGCCTAGTCTATACCTGATAAACTAA[A/T]AT CATACATAGAGAAAATGGTCATTTGGTTGAGTTTC GGTACATGCTGAGATGGTTGCACTTCGGTGCATC TGCTTTGCTTCCATCACATCATAATGTCT |
| 136H5_3M5_7601 | T | C | GCTGCTTGTAGCGTCCCCCATGGCACCTG[T/C]G AAGAGGTTTTCGGCCACAGAGAAGGGGAAGGCT C |
| cfn1288811 | T | G | AAAATTACTTTTCACGCGCTTCGTTGGTCTGACA GTGCGAGCATAATTTTACTTTTTCTCAGTTTTACT TAATTTGGTTAACCAAATCCTTTTTGATTTT[T/G]A ACTAGAAAACCGAATGTCAAACATTGTGCAAATTT GGAAACTGAAACTGAAACCAAAAACCTAAAAAAA TGATTAGTTTGTTTTTTTGTTCTTGTTTCG |
| 136H5_3M5_89176 | A | G | gtatttCTTAGGATTTTCTCACCGGCATCTCC[A/G]TT TTTTGAGCAAGAGTATTTAAGGATGGTAGGC |
| 136H5_3M5_89263 | C | T | AACAAAGATGCTAGTAAGAACATGAACCTAGTTG CTCATTTTTAACAACAATTGCCCACCAACCTGACA TGCTCTTCCCATGTTCTTTTTTTGCTCAAAA[C/T]A GAGATGCTAGTCCAAATATTTTTCTAGTTGCTTAC ATTTTAAACAACAATTGCCTACCATCCTTAAATAC TCTTGCTCAAAAAACGGAGATGCCGGTGA |
| 136H5_3M5_138211 | T | A | AATACAGACTGGGTGCAAAGCCAAGATGAT[T/A]G TAAAATTGATTGATGGCCGTTGGGAGGT |
| cfn0556874 | C | T | TGTAAAGAAGCTTAACCAGGAAAGCTATCAGGGC CATAGGGAATGGCTGGTTAGTGACAATTTTGCCT GCTGGAAATGGGATTTCTTGTTTATTTCAGTT[C/T] TGCATTGTGTCTGACATGCTCTTTCTTTTGGGCG CAGGCTGAAGTGAATTACCTTGGACAACTATCGC ACCCGAATCTTGTAAAGCTCGTTGGGTACTGT |
| 136H5_3M5_64154 | T | C | TGGCGGAGCTGGGGCTGTTCCTCCTACGCAGGC GAAACTTCGCCGCGATAAA[T/C]GGAACTATCATC AGGTTCCCCGATGATCCATACG |
| 136H5_3M5_68807 | A | G | ACAAGCAACCGAGACAAGTTGCTCTTAATTATCT GTGCGT[A/G]CACCTCTAAGTCTTAACCTGACGTA ACCAACCAACCGTGT |
| 136H5_3M5_77916 | A | G | GATGGTTACAAGGCATGCATAGCAAGTAGAGTTA ACTTATCAAGTTATT[A/G]GTATTTTTCTTCTGTGG TACTTAGAGTCTAMAGCTTGAGC |
| cfn1246088 | A | C | AATGGAAGCTGATGTGCGTTAGCGATAAAGCAAC AGCGATAACGACGCATGGATCACCATGCTACTTG GGGAAGCAGGGACATCTGATGAGCCAGCATAC[A /C]CCCAGATATGTGTCTTTCCAAATTCCACGTCC CAACAGATGAGCTATAAATTAATGCCACCTTCCT CCTACAGCTAAATACTCCATCCGTTTCATAATGT |
| cfn1287194 | G | A | CAGAGGCATTCGTGAATTGGGCGAAATCAGAAG CAAGGAGCAGCGATGTTCAGCGCAGAAGGCACT GGGAGGGGATTCCAGGGAGGCTGCCCACCAGC CC[G/A]CCATCAGATACGGAGGAGGTGGATCCAT GGCCCTACCTGTGTCCTGCGCCGAATCTGGACT GTGGTAACTACAGCGCCTGAATCTAGAGGTTCAG CCTGG |
| cfn1258380 | A | C | GATCCATCTCCCTTAATAATTTTGCTATTGGTATT GGGTATGGACATCTGAAGTGAAGGTTACGGCCG ATTTATAGGAGTGATAGCACCACACAATTCAT[A/C ]AGAGCATCTGCAATAGATGAGTAGATGTAAAACT ACTTAACTTTTACATCTCCGGGCCTAAAAACGCAT CTGTAATAAGATAATGTAGATGTAAAGAAAA |
| IWB72107 | A | G | CGACGACGACGAGGATGCCGAGTTTGATGACAT GGAGGATTATATCGACG[A/G]CGCGGACTGGGAC GCCGACATGTATGATGATGTGTTCGATGTCTGAA GGA |
| BS00090770 | C | T | TAGCCGTAGGTCGTAGCACATAGCCGTTTA[C/T]G TAATGCATAGTTGTCCGAAGGAATGTTTC |
| cfn1239345 | A | G | TAACCTGGGGCTTCTTTTTCTCCCTATAATATGG [A/G]CTGCCCTTTTAAGAAGGAACTGCAGCGAGG GTGCA |
| RFL29_S2 | A | G | CTATTCGATGGGCGTGTTTTAATTAACCGGGCAA CTCTCTTCTTCTTAATCA[A/G]TGAAAATGGCAAGT CTTTTACCTCGTTTCAAAAAGAGTTAAATGCACTG GAGGTCCTAAAGGTTTCG |
| RFL29_S4 | C | T | CATGCGGCGGGGGCCCTGCGGCAATGATGACTC CATGAGGGTGG[C/T]TTCGGCGGACGATGCTCTC CGCAGGTTGCACTGGACTAGCTTGGTGTGAGGC TTGCAACTTTCTCCTGTGATGCTCATCAACAAAAT |
| cfn0917304 | G | T | GACTACGCGTTCCTCCCGGTGGTGGCGCTCTAC CC[G/T]TTGTGTTGCCTTTCTCCAAGCAGTTGTGC CCTTCG |
| cfn0919993 | G | T | ATATCTTTACAAGTCATCGACTTACATGCTTCTTT[ G/T]TATTATATGCACCTATGCAGTACTTGTTAATG GGT |
| cfn0920459 | C | G | GATGATATAACCGTAGCCAAGGAAGCCCAGATTT T[C/G]TTCTGTGTATCTATAGGAGCTTAATTAGGA GGAGG |
| cfn0915987 | G | T | AAGTCTGCCATCCAGATCATTACCCAACGGCCAA T[G/T]GAGCCATGAGGTTTGCCTCGTTGCACGTTT TGGCT |
| cfn0920253 | A | C | GCAACAAAGCTGGTCATCCAAACATTTACATCGT T[A/C]GGCAGGCTTTCCGCCCAAACCATGCGGCC GACCTG |
| cfn0448874 | C | T | TATGTAAAACCTCTTTGTTTCTAAATAGCTGCGGC [C/T]CGCTACCTAAATTTATGTTGAACCTAGAGGC ACCC |
| cfn0923814 | A | C | GTTCGGCAGAATCCAAGTCGCAAATGTAAGGTCA G[A/C]AAATGAATGATGATCATGATAATGAAAATC ATAAG |
| cfn0924180 | A | G | ACGTATGGAGCTTCCTCTTTTCATCATGCACCATT [A/G]TGATCTCCCTCTTATTTTGTCTGAAGCCATTC ATG |
| cfn0919484 | A | G | AGGTCATGAAAATGCAAGTGGCGAATCTTATCTC T[A/G]TTATACCATTTGGCAAAACAAAGGCGAGAG TTCTG |
| TaContig158085_61_BS00011513 | A | G | AGTAATTACAACCTTGGCGGCATTTCCAAG[A/G]A CTTCTTTGTCTGCTTTGTCCAGGGACAGT |
| cfn0864865 | C | T | CGGTTTATCTGTTATCATTGTGCACCAGGAAAAC C[C/T]GACTCCTGAGTTCAAAAATATGTTGATTCTA TAAA |
| EXCALIBUR_C96134_152 | T | C | GGCTTAAGGGAGACTCTGGTGACACCATGTAACT T[C/T]ACGGCCACGCACCACCCGTTGGAGTTTGA CAGTTC |
| cfn3133296 | A | G | CTCCCCTGCCAAACCCAAGTGTCCCTCCTTGAGT C[A/G]CAAAGTTGTACCACAGTGGTGGATTGCAA CAGATT |
| LWE1_chr6B_485210_Rf4S | G | T | ACTGGGAACTGCGCTACACCTTCTACGTGGATAA TGGCGACTKCGACGAGCTAMGGGCGTCTGGCA CGATGACAGTAATGGGATGTTGTGCTACTGGCT[ G/T]GGTGACGATCTCTACAAGTATGACACCAGTA AGAAGGGTCAACAACTGACCGCCGATAGTGTCTT GGAGTGGGACCATCGGATCCCCCTGCCGGCAAC TA |
| LWE1_chr6B_11287944_Rf4S | A | G | CTATAAGGAAAGCATATCACTGTAAACAGTATACT ATGACAGAAACAAAGAGTACACAGCATTTTCCCA GGAATATATAATATATACTTCATTTACTGGT[A/G]G TTGATGTGTAGTCCTAAACAGAAGATGAAGGCTA TGCTTTCAATATCAAAAAGCGAAAATAAACAGAAT AACCAGCATAGTTGCAGTAATTACAATTTG |
| LWE1_chr6B_19775886_Rf4S | A | G | TAATTTCTATATACACACATACAACATGTGCACAT ACAATGCGTACGTGGCAAGCAGATCATGACAGG |
| | | | TTGGCGGACGCGGTTCTCCGCCGCGGACGAC[ A/G]CCAGTGCCGGCGCCCTTCATCTCGGCCCGA AATTTGGCGGAGAGTGCGTTTCTCCCGGCTGCG GGAGCCGTGGGCCAAAAGAGGCCCATGCTGACA GCCA |
| LWE1_chr6B_28157776_Rf4S | A | C | TTKATAGTAAAACCAAGCGTACAGAATTAATTTTA GAGCAAACAAAATACTATAATAGAAGTCAAGCCT TGACGCATAAGGTGAAAGCCTGAACCGACG[A/C] TAGTAATCAGTAAGCAGATCGTTTCTAAATAAGAT GATGAATGGTTTATGTTTTCCCGCTGTTAGAATAT TGTTCAGCAAACAGCATAGCTAGGACTGTT |
| RFL46_S2 | A | G | ATTGATGCGCTGTGCAAGGCCAGAGCTATGGAC AAAGCA[A/G]AGTTGTTCCTCCGGCAGATGATTGA TAAAGGTGTTC |
| LWE1_chr7B_658281643_Rf7 | T | G | GAACAATCTCCCCCTACGATTGACTGACGACGAC GAGATCCCACAGTCAAGCCCTCCATTTTCCTCAG AAAACTCTAACGATTCTTACACACGCACCAAA[T/G ]CCGTCACAAGTTTACAGACCCCTGGCATGGATG CACGCACGGTGCAGCCAGCCGGCCCAGGATTTT CATACGTTTGCTATACGTTACGTCGAGAGGGAGT |
| LWE1_chr7B_711539100_Rf7 | C | A | TCAATTCCTTGTTGTCCTTCTTCAGTTCCTCGTTG TGCTTGGCCAGCTTCCTTTTGCCCTCCACCAGCA CCTCGTTCCTCTTCTCCAGATAATCAATCCT[C/A] ATCTTTTTTTTCTCATCGAGCTCCGTCGCCAACTT TCCTTTCTTCTCCACCAGCGCATTTTTTGCCTTTT CCAGATCTGCAATATCTGTTCCCTTTTTTT |

The Examples below are given for illustration purposes only.

### EMBODIMENTS

1. A wheat plant restorer of fertility of *T. timopheevii* CMS cytoplasm, wherein the plant comprises at least Rf1, Rf3, and Rf4s loci.
2. The wheat plant according to Embodiment 1, wherein said Rf1 locus is located within the chromosomal interval between SNP markers cfn0522096 of SEQ ID NO:3 and cfn05277067 of SEQ ID NO:9.
3. The wheat plant of Embodiment 2, wherein said Rf1 locus is characterized by the presence of one or more of the following SNP restorer allele(s):

| SNP# | Marker Name | Marker SEQ ID NO: | Restorer Allele |
|---|---|---|---|
| SNP1 | cfn0523109 | 1 | A |
| SNP2 | 276I13_96B22_97797 | 2 | C |
| SNP3 | cfn0522096 | 3 | C |
| SNP4 | cfn0527763 | 4 | C |
| SNP5 | 104A4_105172 | 5 | TG |
| SNP6 | 104A4_105588 | 6 | A |
| SNP7 | cfn0373248 | 7 | T |
| SNP8 | cfn1 097828 | 8 | C |
| SNP9 | cfn0527067 | 9 | A |
| SNP10 | cfn0528390 | 10 | G |
| SNP11 | BWS0267 | 11 | A |
| SNP12 | cfn0527718 | 12 | T |
| SNP13 | cfn0524469 | 13 | G |
| SNP14 | cfn0524921 | 14 | G |
| SNP15 | cfn1122326 | 15 | C |
| SNP16 | RFL79_S7 | 16 | G |

4. The wheat plant according to any one of Embodiments 1 to 3, wherein said Rf1 locus is characterized by the presence of at least a nucleic acid of SEQ ID NO: 64 or a nucleic acid encoding an amino acid sequence having at least 95% identity, preferably 96%, 97%, 98%, 99% or 100% identity to SEQ ID NO:64.
5. The wheat plant according to any one of Embodiments 1 to 4, wherein the Rf3 locus is located within the chromosomal fragment between SNP markers cfn1249269 of SEQ ID NO:19 and BS00090770 of SEQ ID NO:42.
6. The wheat plant according to Embodiment 5, wherein said Rf3 locus is characterized by the presence of one or more of the following SNP restorer allele(s):

| SNP# | Marker Name | Marker SEQ ID | Restorer Allele |
|---|---|---|---|
| SNP17 | cfn1252000 | 17 | A |
| SNP18 | IWB14060* | 18 | G |
| SNP19 | cfn1249269 | 19 | G |
| SNP20 | 219K1_166464 | 20 | T |
| SNP21 | 219K1_158251 | 21 | G |
| SNP22 | 219K1_111446 | 22 | A |
| SNP23 | 219K1_110042 | 23 | T |
| SNP24 | 219K1_110005 | 24 | C |
| SNP25 | 219K1_107461 | 25 | A |
| SNP26 | 219K1_99688 | 26 | T |
| SNP27 | 219K1_37 | 27 | C |
| SNP28 | cfn1270524 | 28 | T |
| SNP29 | 136H5_3M5_7601 | 29 | T |
| SNP30 | cfn1288811 | 30 | G |
| SNP31 | 136H5_3M5_89176 | 31 | A |
| SNP32 | 136H5_3M5_89263 | 32 | T |
| SNP33 | 136H5_3M5_138211 | 33 | T |
| SNP34 | cfn0556874 | 34 | C |
| SNP35 | 136H5_3M5_64154 | 35 | C |
| SNP36 | 136H5_3M5_68807 | 36 | G |
| SNP37 | 136H5_3M5_77916 | 37 | A |
| SNP38 | cfn1246088 | 38 | A |
| SNP39 | cfn1287194 | 39 | G |
| SNP40 | cfn1258380 | 40 | A |
| SNP41 | IWB72107* | 41 | A |
| SNP42 | BS00090770 | 42 | T |
| SNP43 | cfn1239345 | 43 | A |
| SNP44 | RFL29_S2 | 44 | G |
| SNP 45 | RFL29_S4 | 45 | C |

7. The wheat plant according to any one of Embodiment 5 or 6, wherein said Rf3 locus is characterized by the presence of a nucleic acid encoding an amino acid sequence having at least 95% identity, preferably at least 96%, 97%, 98%, 99% or 100% identity to an amino acid selected from the group consisting of SEQ ID NO: 70, SEQ ID NO: 71 and SEQ ID NO:72.
8. The wheat plant according to any one of Embodiments 1 to 7, wherein said Rf4s locus is located within the chromosomal interval between SNP markers TaConting158085_61_BS0001 1513 of SEQ ID NO:46 and cfn0864865 of SEQ ID NO:47.
9. The wheat plant according to any one of Embodiments 1 to 8, wherein said Rf4s locus comprises any *Ae. Speltoides* SNP on the short arm of the chromosome 6B on the area ranging from 0 to 32 334 597 bases according to IWGSC V1 reference, preferably from the area ranging from 0 to 29 782 272 bases according to IWGSC V1 reference.
10. The wheat plant of Embodiment 8 or 9, wherein said Rf4s locus is characterized by the presence of one or more of the following SNP allele(s):

| SNP# | Marker Name | Marker SEQ ID | Restorer Allele |
|---|---|---|---|
| SNP46 | TaContig158085_61_BS00011 513 | 46 | T |
| SNP47 | cfn0864865 | 47 | G |
| SNP48 | EXCALIBUR_C96134_152 | 48 | C |
| SNP49 | cfn3133296 | 49 | G |
| SNP50 | LWE1_chr6B_485210_Rf4S | 50 | T |
| SNP51 | LWE1_chr6B_11287944_Rf4S | 51 | G |
| SNP52 | LWE1_chr6B_19775886_Rf4S | 52 | G |
| SNP53 | LWE1_chr6B_28157776_Rf4S | 53 | C |

11. The wheat according to any one of Embodiments 1 to 10, wherein the plant also comprises Rf7 and/or 6R locus.
12. The wheat plant according to Embodiment 11, wherein the Rf7 locus is located at most 10 cM from SNP marker cfn0919993 of SEQ ID NO:55.
13. The wheat plant according to Embodiment 11 or 12, wherein said Rf7 locus is characterized by the presence of one or more of the following restorer SNP allele(s):

| SNP# | Marker Name | Marker SEQ ID | Restorer Allele |
|---|---|---|---|
| SNP54 | cfn0917304 | 54 | T |
| SNP55 | cfn0919993 | 55 | G |
| SNP56 | cfn0920459 | 56 | C |
| SNP57 | cfn0915987 | 57 | G |
| SNP58 | cfn0920253 | 58 | A |
| SNP59 | cfn0448874 | 59 | T |
| SNP60 | cfn0923814 | 60 | C |
| SNP61 | cfn0924180 | 61 | G |
| SNP62 | cfn0919484 | 62 | G |
| SNP64 | LWE1_chr7B _658281643_Rf7 | 263 | G |
| SNP65 | LWE1_chr7B _711539100_Rf7 | 264 | A |

14. The wheat plant according to any one of Embodiments11 to 13, wherein the Rf7 locus is characterized by the haplotype "T", "G", "C", "G", "A", "T", "C", "G", "G", "G" and "A" and of the SNP54 to SNP62 and SNP64 to SNP65 restorer alleles respectively as described in the Table of Claim 13.
15. The wheat plant according to any one of Embodiments 11 to 14, wherein it further includes the 6R locus, said 6R locus being located on chromosome 6R and within the chromosomal interval between 48.9 cM to 114.8 cM.
16. The wheat plant according to any one of Embodiments 11 to 15, wherein said 6R locus is characterized by the presence of the following restorer SNP allele:

| SNP# | Marker Name | Marker SEQ ID | Restorer Allele |
|---|---|---|---|
| SNP63 | RFL46_S2 | 63 | A |

17. The wheat plant of any one of Embodiments 1 to 16, wherein representative alleles of Rf1, Rf3, and Rf4s is provided by the seed sample chosen amongst NCIMB 43746 and NCIMB 43747.
18. The wheat plant according to any one of the Embodiments 1 to 17, wherein said wheat plant is alloplasmic and comprises the *T. timopheevii* cytoplasm.
19. A method for producing a wheat hybrid plant comprising the steps of:
- crossing a sterile female comprising the *T. timopheevii* cytoplasm with a fertile male wheat plant according to any one of Embodiments 1 to 18;
- collecting the hybrid seed;
- optionally detecting the presence of *T.timopheevii* cytoplasm, and/or at least three of the Rf locus chosen amongst Rf1, Rf3, Rf4s, Rf7 and 6R in the hybrid seed;
- optionally detecting hybridity level of the hybrid seeds.
20. The method of Embodiment 19, further comprising after step b), a step of detecting the presence of *T. timopheevii* cytoplasm, and/or the Rf1, Rf3, and Rf4s restorer alleles in the hybrid seeds.
21. The method of Embodiment 20, further comprising a step of detecting the presence of loci Rf7 or 6R in the hybrid seeds.
22. A method for producing a wheat hybrid seed comprising the steps of:
a. crossing a first and a second wheat plant according to any one of Embodiments 1 to 18;
b. collecting the hybrid seed;
c. optionally detecting hybridity level of the hybrid seeds.
23. The method of Embodiment 22, wherein the fertility score of the obtained wheat plant has a fertility score higher than the parent wheat plant.
24. A wheat hybrid plant as obtained by the method of Embodiments 19 to 23.
25. A method of identifying a wheat plant according to any one of Embodiments 1 to 18, wherein said wheat plant is identified by detecting the presence of at least one restorer allele within one or more of Rf1, Rf3, Rf4s, Rf7 and 6R loci, preferably within the three Rf1, Rf3 and Rf4s loci.
26. A method for producing the wheat plant restorer of fertility of any one of Embodiments 1 to 18, said method comprising the following steps:
a. providing a first wheat plant comprising one or two restorer allele selected among Rf1, Rf3 and Rf4s restorer alleles,
b. crossing said first wheat plant with a second wheat plant comprising one or two restorer alleles selected among Rf1, Rf3 and Rf4s restorer alleles, wherein Rf1, Rf3 and Rf4s restorer alleles are represented at least once in the panel of restorer alleles provided by the first plant and the second plant,
c. collecting the F1 hybrid seed,
d. obtaining homozygous plants from the F1 plants,
e. detecting the presence of the Rf1, Rf3 and Rf4s restorer alleles in the hybrid seed and/or at each generation.
27. A method for producing the wheat plant restorer of fertility, said method comprising the following steps:
e. providing a first wheat plant comprising at least Rf1, Rf3 and Rf4s restorer alleles according to any one of Embodiments 1 to 18,
f. crossing said first wheat plant with a second wheat plant,
g. collecting the F1 hybrid seed,
h. obtaining homozygous plants from the F1 plants,
i. optionally detecting the presence of the Rf1, Rf3 and Rf4s restorer alleles in the hybrid seed and/or at each generation, and optionally further detecting the presence of Rf7 and/or 6R restorer alleles in the hybrid seed and/or at each generation.
28. A method for producing a wheat plant restorer of fertility, said method comprising the following steps:
d. crossing a first wheat plant according to any one of Claims 1 to 18 with a second wheat plant; thereby obtaining a F1 hybrid plant;
e. backcrossing said F1 hybrid with the second wheat plant;
f. selecting the wheat plant restorer of fertility among the wheat plant obtained in step b) by detecting the presence of at least Rf1, Rf3 and Rf4s restorer alleles, and optionally further detecting the presence of Rf7 and/or 6R restorer alleles.
29. The method for producing a wheat plant restorer of fertility according to Claim 28, said method further comprises one or more step of backcrossing the wheat plant selected by detecting the presence of at least Rf1, Rf3 and Rf4s restorer alleles, and optionally by further detecting the presence of Rf7 and/or 6R restorer alleles.
30. The method for producing a wheat plant restorer of fertility according to Claim 28 or 29, wherein the second wheat plant is an elite wheat line.

### EXAMPLES:

### Example 1: Origin, restoring efficiency and breeding of the locus Rf4s from Aegilops speltoides.

Rf4 is located on the 6B chromosome of the accession R113, which also carry the restorer gene Rf1, and is partially restoring the fertility of alloplasmic wheat with *T. timopheevii* cytoplasm (Maan et al;, 1984).

L13 is a line derived from R113 and carry only Rf4 (Australian Grain Genebank 90819). The presence of Rf4 has been confirmed via a QTL identification located on 6BS in a mapping population of 117 F2 individuals of a cross "CMS line/L13" (Data not shown). Locus on 6BS with a pvalue = 1.08E⁻¹³, explains 70.5% of the total variance).

GSTR435 is a *Ae. speltoides* introgression line with Lr36 (Pedigree: Neepawa/Line 2-9-2(Neepawa*5/Aegilops speltoides 2-9)//3*Manitou; USDA, E84018). The introgression is located at the distal part of the short arm of chromosome 6B (Dvorak J and Knott DR, 1990).

GSTR435 is partially restoring the fertility of alloplasmic wheat with *T. timopheevii* cytoplasm and this partial restoration of fertility is higher than that of L13. See Table 8

**Table 8: Fertility level expressed as the average number of kernels per spikelet of F1 plants (cross sterile CMS line/GSTR435 or L13). F1 plants = number of individual F1 plants, σ = standard deviation, x̅ = average in kernels per spikelet**

| Genotype | F1 plants | σ | x̅ |
|---|---|---|---|
| GSTR435 | 10 | 1.0 | 1.7 |
| L13 | 12 | 0.4 | 0.8 |

A mapping population has shown that the restoring locus of GSTR435 is located on the distal part of the short arm of chromosome 6B (Data not shown). This mapping population was made of a F2 population of 94 individuals of a cross "CMS line/GSTR435" (Locus on 6BS with a pvalue = 3.15^{E-12}, explains 68.4% of the total variance).

This new restorer locus has been named Rf4s, in opposition to the Rf4 locus present in R113 and L13 of *T. timopheevii* origin.

LGWR20-0485 is an alloplasmic restorer line developed by Limagrain through pedigree breeding from a cross between GSTR435, Rf1 and Rf3strong donors and elite lines. LGWR20-0485 is a winter wheat type agronomically adapted to the cultivation in Western Europe and is homozygous for the restorer alleles Rf1, Rf3 and the introgression from GSTR435 carrying Rf4s (Table 9). Sister lines with the same haplotype have been derived from the same initial cross. The presence of the locus Rf4s is revealed by the use of the KASP LWE1_chr6B_28157776_Rf4 and LWE1_chr6B_11287944_Rf4S.

**Table 9: Haplotypes for the loci Rf1, Rf3strong, Rf4s and the cytoplasm of the maintainor elite line Apache and of the restorer line LGWR20-0485**

| Locus | Rf1 | Rf3 strong | Rf4s | Cytoplam |
|---|---|---|---|---|
| Marker | RFL79_S7 | RFL29_S4 | LWE1_chr6B_28157776_Rf4 | ORF279_S4 |
| APACHE | A | T | A | G |
| LGWR20-0485 | G | C | C | C |

### Example 2: Characterization of the genomic region containing Rf4 Aegilops speltoides genetic determinants

Two strategies have been used to determine Rf4s genomic region from *Ae. speltoides.* A F2 mapping population to perform fine mapping and sequencing data do dertermine the *Ae. speltoides* introgression size from the GSTR435 donor line.

First, a F2 mapping population segregating for Rf4 GSTR435 x Manenick_CMS encompassing 94 individuals was phenotyped and genotyped with 18100 SNP markers using Limagrain's internal genotyping platform.

Fertility tests were conducted indoors under controlled growth conditions, either in growth chambers or in greenhouses, enabling normal fertility of the tested wheat plants. The fertility scores indicated have been calculated by dividing the total number of seeds threshed from a spike by the number of counted spikelets. Whole genome QTL analysis was conducted on F2 plants using a Composite Interval Mapping approach (internal tool) and internal genetic consensus map.

Rf4 was first mapped on the short arm of the chromosome 6B between 6 cM and 43 cM on Limagrain's internal consensus map and physically delimited by SNP markers TaContig158085_61_BS00011513 and cfn0864865. These two SNP markers delimit the largest possible interval defined by the three mapping populations.

In a second step, the locus was fine-mapped by screening 1811 and 3142 F3 lines from GSTR435 x Manenick_CMS derived from F2 plants heterozygous at the locus. Phenotyping and analysis of recombinant plant progenies within the interval redefined a smaller mapping interval between 6 and 36 cM delimited by EXCALIBUR_C96134_152 and cfn3133296 SNP markers. Using this new QTL analysis, we concluded that the gene could be from the start of the 6B chromosome to the cfn3133296 marker so from 0 to the physical position 29 782 272 (position reference IWGSC V1).

Secondly, we used sequencing data do determine the GSTR435 *Ae. speltoides* introgression size containing RF4 locus. Due to the wheat genome size, we performed internal partial sequencing using Exome capture approach. Exome sequencing strategy is commonly used in wheat to detect SNP and highlights wild-relative introgression (Hu et al., 2019), but any whole genome sequencing strategy can give access to the same information.

By computing reads sequences coverage variations inside exons between different non Rf4 wheat lines and the Rf4 GSTR435 line, it has been confirmed that the GSTR435 contain an alien introgression (*Ae. speltoides*) on the short arm of the 6B chromosome. We estimated the *Aegilops speltoides* introgression position from start 0 to 32 334 597 bp on 6B (physical position reference IWGSC V1).

Finally, 377 polymorphic SNP specific from GSTR435 6B Ae. speltoides introgression have been extracted and are usable to follow and identify the Rf4s locus. From the 377 polymorphic SNP, 4 have been converted into Kaspar markers to follow the Rf4s introgression:
- LWE1_chr6B_485210_Rf4S,
- LWE1_chr6B_11287944_Rf4S,
- LWE1_chr6B_19775886_Rf4S, and
- LWE1_chr6B_28157776_Rf4S.

### Example 3: Origin, restoring efficiency and breeding of the T4BS 6BL 6RL rye introgression

The long arm of chromosome 6R of rye from addition lines is restoring the fertility of alloplasmic wheat with *T. timopheevii* cytoplasm (Curtis and Lukaszewski, 1993).

4 translocation lines with 6RL have been created and are available upon order at the Wheat Genetics Resource Center of the Kansas State University They contain three different events of translocation between 6RL and wheat chromosomes:
- TA5030 (KS92WGRC17, PI592729) T6BS·6BL-6RL (Sebesta et al., 1997)
- TA5031(KS92WGRC18, P1592730) T4BS·4BL-6RL (Sebesta et al., 1997)
- TA5032 (KS92WGRC19, PI592731) T4BS·4BL-6RL (Sebesta et al., 1997)
- TA5041 (KS93WGRC28, PI583794) T6BS·6RL, descendant of TA2929 (TAM104, Friebe et al., 1995)

The three radiation induced chromosomal translocations T6BS·6RL, T6BS·6BL-6RL and T4BS·4BL-6RL can restore partially the fertility of alloplasmic wheat with *T. timopheevii* cytoplasm (Table 10). The 6RL arm has a proximal region with homoeology to the wheat group 6 chromosome, one interstitial region with homoeology to the long arms of the wheat group 3 chromosomes and a distal region with homoeology to the long arms of the wheat group 7 chromosomes (Devos et al., 1993). It is consequently highly unlikely that the translocated 6RL chromosome piece in T4BS·4BL-6RL may recombine with the group chromosome.

**Table 10: Fertility level expressed as the average number of kernels per spikelet of F1 plants (cross sterile CMS line/TA2929 or TA5030 or TA5031). F1 plants = number of individual F1 plants, σ = standard deviation, x̅ = average of kernels per spikelet**

| Genotype | F1 plants | σ | x̅ |
|---|---|---|---|
| TA2929 | 16 | 0.46 | 1.04 |
| TA5030 | 51 | 1.01 | 1.14 |
| TA5031 | 64 | 0.87 | 1.69 |

LGWR17-0160 is an alloplasmic restorer line developed by Limagrain through pedigree breeding from a cross between TA5031, Rf1 and Rf3 donors and elite lines.

LGWR17-0160 is a winter wheat type line agronomically adapted to the cultivation in Western Europe and is homozygous for the restorer alleles Rf1, Rf3 and the introgression T4BS·4BL-6RL from TA5031 (Table 11, the T4BS·4BL-6RL translocation being called "6RL").

The T4BS·4BL-6RL translocation is revealed by the allele "A" for the KASP marker RFL46_S2, the allele "G" of this marker indicates the absence of the translocation and consequently the absence of the capacity of fertility restoration. The accession TA5041 is equally carrying the restorer allele "A" for the KASP marker RFL46_S2.

**Table 11: Haplotypes for the loci Rf1, Rf3, the introgression T4BS·4BL-6RL (coded 6R) and the cytoplasm of the maintain or elite line Apache and of the restorer line LGWR17-0160**

| Locus | Rf1 | Rf3 | 6RL | Cytoplasm |
|---|---|---|---|---|
| Marker | RFL79_S7 | RFL29_S4 | RFL46_S2 | ORF279_S4 |
| APACHE | A | T | G | G |
| LGWR17-160 | G | C | A | C |

The TA5031 should have the T4BS·4BL-6RL translocation. But according to the dominant profiles of the markers and the fact that the RFL46_S2 is "diagnostic" we think that this donor is T6BL-6RL translocated. We noticed these dominant profiles on the 6B from 48.9cM to 114.8cM (=end of the chromosome).

### Example 4: New Rf alleles combinations for a full fertility restoration of CMS hybrids

It is largely admitted that the full fertility restoration of the cultivated CMS hybrid can only be reached by the cumulative effect of several Rf loci with major effect, possibly with the combined help of modifier gene(s) that may help enhance the overall fertility expression.

A minimum of three Rf genes would need to be bred together in a restorer line to create a timely and geographically stable restoration of fertility of the F1.

The use of molecular markers tightly linked to the respective Loci is therefore necessary as the fertility scores of the restorer line alone would not suffice in creating a combination of three Rf alleles.

The following example demonstrates the impossibility to create restorer line, with the *Triticum timopheevii* cytoplasm, homozygous at three loci or more without using molecular markers strictly linked to the genes.

This applies to any breeding method used as for instance doubled haploid, pedigree breeding, single seed descent, backcross.

Out of the 27 possible different haplotypes created through the generations, 17 could lead to a full fertility of the restorer line misleading the breeder into creating fully fertile restorer lines not containing all the three restoring alleles (Table 12).

**Table 12: Estimated level of fertility restoration for every individual haplotype of the combination between restoring alleles (Rf) and non-restoring alleles (rf) of the 3 loci Rf1, Rf3 and Rf4s in alloplasmic restorer lines.**

| **Restorer alleles** | **Locus Rf1** | **Locus Rf3strong** | **LocusRf4s** | **Fertility** |
|---|---|---|---|---|
| 0 | rf1/rf1 | rf3/rf3 | rf4s/rf4s | 0% |
| 1 | rf1/**Rf1** | rf3/rf3 | rf4s/rf4s | 25-50% |
| 1 | rf1/rf1 | rf3/**Rf3** | rf4s/rf4s | 25-50% |
| 1 | rf1/rf1 | rf3/rf3 | rf4s/**Rf4s** | 25-50% |
| 2 | **Rf1/Rf1** | rf3/rf3 | rf4s/rf4s | 50-75% |
| 2 | rf1/rf1 | **Rf3/Rf3** | rf4s/rf4s | 50-75% |
| 2 | rf1/rf1 | rf3/rf3 | **Rf4s/Rf4s** | 50-75% |
| 2 | rf1/**Rf1** | rf3/**Rf3** | rf4s/rf4s | 50-75% |
| 2 | rf1/**Rf1** | rf3/rf3 | rf4s/**Rf4s** | 50-75% |
| 2 | rf1/rf1 | rf3/**Rf3** | rf4s/**Rf4s** | 50-75% |
| 3 | **Rf1/Rf1** | rf3/**Rf3** | rf4s/rf4s | full |
| 3 | **Rf1/Rf1** | rf3/rf3 | rf4s/**Rf4s** | full |
| 3 | rf1/**Rf1** | **Rf3/Rf3** | rf4s/rf4s | full |
| 3 | rf1/rf1 | **Rf3/Rf3** | rf4s/**Rf4s** | full |
| 3 | rf1/**Rf1** | rf3/rf3 | **Rf4s/Rf4s** | full |
| 3 | rf1/rf1 | rf3/**Rf3** | **Rf4s/Rf4s** | full |
| 3 | rf1/**Rf1** | rf3/**Rf3** | rf4s/**Rf4s** | full |
| 4 | **Rf1/Rf1** | **Rf3/Rf3** | rf4s/rf4s | full |
| 4 | **Rf1/Rf1** | rf3/**Rf3** | rf4s/**Rf4s** | full |
| 4 | rf1/rf1 | **Rf3/Rf3** | **Rf4s/Rf4s** | full |
| 4 | rf1/**Rf1** | **Rf3/Rf3** | rf4s/**Rf4s** | full |
| 4 | rf1/rf1 | **Rf3/Rf3** | **Rf4s/Rf4s** | full |
| 4 | rf1/**Rf1** | rf3/**Rf3** | **Rf4s/Rf4s** | full |
| 5 | **Rf1/Rf1** | **Rf3/Rf3** | rf4s/**Rf4s** | full |
| 5 | **Rf1/Rf1** | rf3/**Rf3** | **Rf4s/Rf4s** | full |
| 5 | rf1/**Rf1** | **Rf3/Rf3** | **Rf4s/Rf4s** | full |
| 6 | **Rf1/Rf1** | **Rf3/Rf3** | **Rf4s/Rf4s** | full |

Nine Rf genes restoring the fertility of the *T. timopheevii* cytoplasm have been identified to date: Rf1 (1A), Rf2 (7D), Rf3 (1B), Rf4 (6B), Rf5 (6D), Rf6 (5D), Rf7 (7B), Rf8 (2D) and Rf9 (6AS) (Tahir and Tsunewaki 1969; Yenet al., 1969; Bahl and Maan 1973; Mukai and Tsunewaki 1979; Wilson and Driscoll 1983; Maan et al., 1985; Du et al., 1991; Sinha et al., 2013; Shahinnia et al. 2020). Individually, all those 9 Rf locus may display different levels of expressivity and their combinations may not prove strictly additive, exemplified by the non-additive effect of the loci Rf4 and Rf1 (Geyer et al., 2017).

Table 13 below shows a series of F1 fertility scorings when using restorer lines with different combinations of 3 to 4 restorer alleles.

**Table 13: Fertility level, expressed as the average number of seeds per spikelet, and seedset, expressed as the average total number of seeds per spike, of F1 plants produced with a series of restorer lines displaying different combination of the restorer loci Rf1, Rf3, Rf3w (Rf3 "weak"), Rf4, Rf4s and 6R. nb = number of individual spikes. σ = standard deviation, x̅ = average.**

| | **FERTILITY** | | | **SEEDSET** | | |
|---|---|---|---|---|---|---|
| | nb | average | σ | nb | average | σ |
| Rf1+Rf3+Rf7 | 119 | 2,84 | 0,51 | 119 | 57,21 | 13,69 |
| Rf1+Rf3+6R | 42 | 2,41 | 0,44 | 42 | 54,14 | 11,31 |
| Rf1+Rf3+Rf4s | 93 | 2,59 | 0,35 | 93 | 59,09 | 10,97 |
| Rf1+Rf3+Rf4 | 7 | 2,08 | 0,45 | 7 | 37,00 | 9,09 |
| Rf1+Rf3+Rf4+Rf7 | 9 | 2,16 | 0,32 | 9 | 49,00 | 7,55 |
| Rf1+Rf3w+Rf4 +Rf7 | 39 | 2,22 | 0,32 | 39 | 41,82 | 6,92 |
| Rf1+Rf3w+Rf7 | 265 | 2,37 | 0,45 | 265 | 53,83 | 11,37 |
| Rf3+Rf7+6R | 18 | 2,62 | 0,22 | 18 | 52,28 | 7,09 |
| Rf3w+Rf4+Rf7 | 10 | 1,80 | 0,48 | 10 | 34,40 | 10,52 |
| Checks | 189 | 2,7 | 0,5 | 189 | 56,9 | 12,1 |

Four combinations of three Rf restoring alleles are either statistically better or equivalent (α = 0.05) to the group of elite lines (indicated as checks), either for the fertility or for the seedset or for both: Rf1+Rf3+Rf7, Rf3+Rf7+6R, Rf1+Rf3+Rf4s and Rf1+Rf3+6R. All the other combinations of three or four Rf alleles are statistically inferior to the group of checks (α = 0.05) for both fertility and seedset (Tables 14 and 15).

**Table 14: t Student test for mean comparison for fertility of F1 plants and checks (average number of kernels per spike). The haplotype column indicates the Rf alleles combination of the restorer line used to produce the F1 plants. α = 0.05**

| **Haplotype** | | | | | | **Mean** |
|---|---|---|---|---|---|---|
| Rf1+Rf3+Rf7 | A | | | | | 2.8360804 |
| checks | A | | | | | 2.7353917 |
| Rf3+Rf7+6R | A | B | C | | | 2.6153608 |
| Rf1+Rf3+Rf4s | | B | | | | 2.5861419 |
| Rf1+Rf3+6R | | | C | D | | 2.4073307 |
| Rf1+Rf3w+Rf7 | | | | D | | 2.3675521 |
| Rf1+Rf3w+Rf4+Rf7 | | | | D | | 2.2183024 |
| Rf1+Rf3+Rf4+Rf7 | | | | D | E | 2.1648731 |
| Rf1+Rf3+Rf4 | | | | D | E | 2.0812417 |
| Rf3w+Rf4+Rf7 | | | | | E | 1.7988889 |

**Table 15: t Student test for mean comparison for seedset of F1 plants and checks (average number of kernels per spike). The haplotype column indicates the Rf alleles combination of the restorer line used to produce the F1 plants. α = 0.05**

| **Haplotype** | | | | | | **Mean** |
|---|---|---|---|---|---|---|
| Rf1+Rf3+Rf4s | A | | | | | 59.086022 |
| Rf1+Rf3+Rf7 | A | B | | | | 57.210084 |
| checks | A | B | | | | 56.888889 |
| Rf1+Rf3+6R | | B | C | | | 54.142857 |
| Rf1+Rf3w+Rf7 | | | C | | | 53.826415 |
| Rf3+Rf7+6R | | B | C | | | 52.277778 |
| Rf1+Rf3+Rf4+Rf7 | | | C | D | | 49.000000 |
| Rf1+Rf3w+Rf4+Rf7 | | | | D | E | 41.820513 |
| Rf1+Rf3+Rf4 | | | | | E | 37.000000 |
| Rf3w+Rf4+Rf7 | | | | | E | 34.400000 |

In Tables 14 and 15, for all variables with the same letter, the difference between the means is not statistically significant.

### BIBLIOGRAPHY

Ahmed et al., 2001. QTL analysis of fertility restoration against cytoplasmic male sterility in wheat. Genes Genet Syst, 76:33-38.
Bahl PN, Maan SS, 1973. Chromosomal location of fertility restoring genes in six lines of common wheat. Crop Sci 13: 317-320.
Bennetzen JL et al., 2012.Reference genome sequence of the model plant Setaria. Nat Biotechnol 30 (6):555-+. doi:10.1038/nbt.2196
Brenchley R, et al., 2012. Analysis of the bread wheat genome using whole-genome shotgun sequencing. Nature 491 (7426):705-710. doi:1 0.1 038/nature11650
Cannarozzi G, et al., 2014. Genome and transcriptome sequencing identifies breeding targets in the orphan crop tef (Eragrostis tef). Bmc Genomics 15. doi:Artn 58110.1186/1471-2164-15-581
Chen JF et al., 2013. Whole-genome sequencing of Oryza brachyantha reveals mechanisms underlying Oryza genome evolution. Nature Communications 4. doi:ARTN 159510.1038/ncomms2596
Cheng SF, Gutmann B, Zhong X, Ye YT, Fisher MF, Bai FQ, Castleden I, Song Y, Song B, Huang JY, Liu X, Xu X, Lim BL, Bond CS, Yiu SM, Small I (2016) Redefining the structural motifs that determine RNA binding and RNA editing by pentatricopeptide repeat proteins in land plants. Plant Journal 85 (4):532-547. doi:10.1111/tpj.13121.
Christensen AH and Quail PH, 1996. Ubiquitin promoter-based vectors for high-level expression of selectable and/or screenable marker genes in monocotyledonous plants. Transgenic Res, May;5(3):213-8.
Christian et al., 1992. Maize polyubiquitin genes: structure, thermal perturbation of expression and transcript splicing, and promoter activity following transfer to protoplasts by electroporation. Plant Mol Biol. 18(4):675-89.
Curtis and Lukaszewski, 1993. Localization of genes in Rye that restore male fertility to hexaploid wheat with timopheevii cytoplasm. Plant breeding, 11:106-112.
Depigny-This D et al., 1992. The cruciferin gene family in radish. Plant Molecular Biology, 20: 467-479.
Du H, Maan SS, Hammond JJ (1991) Genetic analyses of male fertility restoration in wheat. III. Effects of aneuploidy. Crop Sci 31:319-322
Fehr WR et al, 1987. Principles of Cultivar Development Vol.1 Theory and Technique. Macmillan, New York..
Fujii S, Bond CS, Small ID (2011) Selection patterns on restorer-like genes reveal a conflict between nuclear and mitochondrial genomes throughout angiosperm evolution. P Natl Acad Sci USA 108 (4):1723-1728. doi:DOI 10.1073/pnas.1007667108.
Geyer M et al., 2016. Distribution of the fertility-restoring gene Rf3 in common and spelt wheat determined by an informative SNP marker. Mol Breeding, 36:167. DOI 10.1007/s11032-016-0592-6.
Götz H et al., 2011.Transgene Expression Systems in the Triticeae Cereals. Journal of Plant Physiology 168, no. 1 : 30-44. doi:10.1016/j.jplph.2010.07.007.
International Brachypodium I (2010) Genome sequencing and analysis of the model grass Brachypodium distachyon. Nature 463 (7282):763-768. doi:10.1038/nature08747
Jacquemin J, et al., 2013. The International Oryza Map Alignment Project: development of a genus-wide comparative genomics platform to help solve the 9 billion-people question. Curr Opin Plant Biol 16 (2):147-156. doi:10.1016/j.pbi.2013.02.014.
Jefferson, R.A., 1987. Assaying chimeric genes in plants: The GUS gene fusion system. Plant Mol. Biol. Report. 5, 387-405. DOI:10.1007/BF02667740
Jia J, et al., 2013. Aegilops tauschii draft genome sequence reveals a gene repertoire for wheat adaptation. Nature 496 (7443):91-95. doi:10.1038/nature12028.
Jones HD, 2015. Wheat Biotechnology: Current Status and Future Prospects. K. Azhakanandam et al. (eds.), Recent Advancements in Gene Expression and Enabling Technologies in Crop Plants, DOI 10.1007/978-1-4939-2202-4_8.
Kay R, et al., 1987. Duplication of CaMV 35S promoter sequences creates a strong enhancer for plant genes. Science 236:1299-1302.
Kawahara Y et al., 2013. Improvement of the Oryza sativa Nipponbare reference genome using next generation sequence and optical map data. Rice 6. doi:Artn 410.1186/1939-8433-6-4.
Kihara, 1951, Genome analysis in Triticum and Aegilops X. Concluding review. Cytologia, 16: 101-123.
Kojima et al., 1997, High-resolution RFLP mapping of the fertility restoration (Rf3) gene against Triticum timopheevii cytoplasm located on chromosome 1BS of common wheat. Genes Genet Syst, 72: 353-359.
Krasileva KV et al., 2013. Separating homeologs by phasing in the tetraploid wheat transcriptome. Genome Biol 14 (6). doi:ARTN R66 10.1186/gb-2013-14-6-r66.
Li et al., 2003. OrthoMCL: Identification of Ortholog Groups for Eukaryotic Genomes. Genome Res. 2003 Sep; 13(9): 2178-2189.
Li H. and Durbin R, 2010. Fast and accurate long-read alignment with Burrows-Wheeler Transform. Bioinformatics, Epub. [PMID: 20080505]
Ling HQ et al., 2013. Draft genome of the wheat A-genome progenitor Triticum urartu. Nature 496 (7443):87-90. doi:10.1038/nature11997
Longin et al., 2012 , Hybrid breeding in autogamous cereals. Theor Appl Genet.: 125:1007-1096. DOI 10.1007/s00122012-1967-7.
Ma ZQ and Sorrells ME, 1995, Genetic analysis of fertility restoration in wheat using RFLP. Crop Sci., 35:1137-1143.
Maan, S. S. Genetic analyses of male-fertility restoration in wheat: isolation, penetrance, and expressivity of Rf genes. Crop Sci. 25, 743-748 (1985).
Mace ES et al., 2013. Whole-genome sequencing reveals untapped genetic potential in Africa's indigenous cereal crop sorghum. Nat Commun 4:2320. doi:10.1038/ncomms3320.
McElroy D et al., 1990. Isolation of an Efficient Actin Promoter for Use in Rice Transformation. The Plant Cell, Vol. 2, 163-171.
Martis MM et al., 2013. Reticulate Evolution of the Rye Genome. Plant Cell 25 (10):3685-3698. doi:10.1105/tpc.113.114553
Mayer KFX, et al., 2014. A chromosome-based draft sequence of the hexaploid bread wheat (Triticum aestivum) genome. Science 345 (6194). doi:ARTN 125178810.1126/science. 1251788
Mayer KFX et al., Conso IBGS, 2012. A physical, genetic and functional sequence assembly of the barley genome. Nature 491 (7426):711-+. doi:10.1038/nature11543.
Melonek, J., Duarte, J., Martin, J. et al. The genetic basis of cytoplasmic male sterility and fertility restoration in wheat. Nat Commun 12, 1036 (2021). https://doi.org/10.1038/s41467-021-21225-0
Mukai Y, Tsunewaki K (1979) Basic studies on hybrid wheat breeding. A new male sterility-fertility restoration system in common wheat utilizing the cytoplasms of Aegilops kotschyi and Ae. Variabilis. Theor Appl Genet 54:153-160.
Ouyang S et al, 2007. The TIGR Rice Genome Annotation Resource: Improvements and new features. Nucleic Acids Res 35:D883-D887. doi:10.1093/nar/gkl976.
Pallavi Sinha P et al., 2013. Genetic analysis and molecular mapping of a new fertility restorer gene Rf8 for Triticum timopheevii cytoplasm in wheat (Triticum aestivum L.) using SSR markers. Genetica, 141: 131-141.
Paterson AH et al., 2009. The Sorghum bicolor genome and the diversification of grasses. Nature 457 (7229):551-556. doi:10.1038/nature07723.
Rathburn and Hedgcoth, 1991. Chimeric open reading frame in the 5' flanking region of coxl mitochondrial DNA from cytoplasmic male-sterile wheat. Plant Mol. Biol., 16:909-912.
Rice P et al., 2000. A. EMBOSS: The European molecular biology open software suite. Trends Genet 16, 276-277, 10.1016/S0168-9525(00)02024-2.
Sakai H, et al., 2013. Rice Annotation Project Database (RAP-DB): An Integrative and Interactive Database for Rice Genomics. Plant Cell Physiol 54 (2):E6-+. doi:10.1093/pcp/pcs183.
Schnable PS, et al., 2009. The B73 Maize Genome: Complexity, Diversity, and Dynamics. Science 326 (5956):1112-1115. doi: 1178534.
Shahinnia F, Geyer M, Block A, Mohler V, Hartl L. Identification of Rf9, a Gene Contributing to the Genetic Complexity of Fertility Restoration in Hybrid Wheat. Front Plant Sci. 2020 Dec 10;11:577475. doi: 10.3389/fpls.2020.577475. PMID: 33362809; PMCID: PMC7758405.
Sinha, P., Tomar, S. M. S., Vinod, Singh, V. K., and Balyan, H. S. (2013). Genetic analysis and molecular mapping of a new fertility restorer gene Rf8 for Triticum timopheevi cytoplasm in wheat (Triticum aestivum L.) using SSR markers. Genetica 141, 431-441. doi: 10.1007/s10709-013-9742-5
Singh SK et al., 2010. Perspective of hybrid wheat research: a review. Indian J Agric Sci 80:1013-1027.
Song and Hedgcoth, 1994. Influence of nuclear background on transcription of a chimeric gene orf256 and cox1 in fertile and cytoplasmic male sterile wheats.Genome, vol.37
Stojalowski S et al., 2013. The importance of chromosomes from the sixth homeologic group in the restoration of male fertility in winter triticale with Triticum tomopheevii cytoplasm. J.Appl. Genetics, 54:179-184.
Tahir, C. M. & Tsunewaki, K. Monosomic analysis of Triticum spelta var. duhamelianum, a fertility-restorer for T. timopheevi cytoplasm. Jpn. J. Genet. 44, 1-9 (1969).
Ch. M. Tahir and K. Tsunewaki, 1971. Monosomic analysis of fertility-restoring genes in triticum aestivum strain p168. Canadian Journal of Genetics and Cytology. https://doi.org/10.1139/g71-003.
Verdaguer et al., 1996. Isolation and expression in transgenic tobacco and rice plants, of the cassava vein mosaic virus (CVMV) promoter. Plant Molecular Biology 31: 1129-1139.
Wang MH et al., 2014. The genome sequence of African rice (Oryza glaberrima) and evidence for independent domestication. Nat Genet 46 (9):982-+. doi:10.1038/ng.3044
Wilson JA, Ross WM. 1962. Male sterility interaction of the Triticum aestivum nucleus and Triticum timopheevii cytoplasm. Wheat Information Service (Kyoto) 14: 29-30.
Wilson JA, Ross WM. 1962. Male sterility interaction of the Triticum aestivum nucleus and Triticum timopheevii cytoplasm. Wheat Information Service (Kyoto) 14, 29-30.
Wilson P, Driscoll Cj, 1983. Hybrid Wheat. Monographs on theorical and applied genetics, Vol. 6, 94-123.
Wilson, 1984. Hybrid wheat breeding and commercial seed development. Plant Breeding Rev., 2: 303-319. 2
Whitford R et al., 2013. Hybrid breeding in wheat: technologies to improve hybrid wheat seed production. Journal of Experimental Botany. Doi:10.1093/jxb/ert333.
Zhou et al., 2005. SSR marker associated with fertility restoration genes against Triticum timopheevii cytoplasm in Triticum aestivum. Euphytica, 141:33-40.
Sebesta EE, Hatchett JH, Friebe B, Gill BS, Cox TS, and Sears RG. 1997. Registration of KS92WGRC17, KS92WGRC18, KS92WGRC19, and KS92WGRC20 winter wheat germplasms resistant to Hessian fly. Crop Sci 37:635.
https://doi.org/10.2135/cropsci1997.0011183X003700020065x
Friebe B, Gill BS, Tuleen NA, and Cox TS. 1995. Crop Sci 35:1237

## Claims

1. A method of identifying a wheat plant restorer of fertility of *T. timopheevii* CMS cytoplasm, wherein said method comprising detecting the presence of at least one restorer allele within one or more of Rf1, Rf3, Rf4s, Rf7 and 6R loci, preferably within the three Rf1, Rf3 and Rf4s loci.

2. The method of Claim 1, wherein said Rf1 locus is located within the chromosomal interval between SNP markers cfn0522096 of SEQ ID NO:3 and cfn05277067 of SEQ ID NO:9.

3. The wheat plant of Claim 1, wherein said Rf1 locus is **characterized by** the presence of one or more of the following SNP restorer allele(s):
| SNP# | Marker Name | Marker SEQ ID NO: | Restorer Allele |
|---|---|---|---|
| SNP1 | cfn0523109 | 1 | A |
| SNP2 | 276I13_96B22_97797 | 2 | C |
| SNP3 | cfn0522096 | 3 | C |
| SNP4 | cfn0527763 | 4 | C |
| SNP5 | 104A4_105172 | 5 | TG |
| SNP6 | 104A4_105588 | 6 | A |
| SNP7 | cfn0373248 | 7 | T |
| SNP8 | cfn1097828 | 8 | C |
| SNP9 | cfn0527067 | 9 | A |
| SNP10 | cfn0528390 | 10 | G |
| SNP11 | BWS0267 | 11 | A |
| SNP12 | cfn0527718 | 12 | T |
| SNP13 | cfn0524469 | 13 | G |
| SNP14 | cfn0524921 | 14 | G |
| SNP15 | cfn1122326 | 15 | C |
| SNP16 | RFL79_S7 | 16 | G |

4. The method according to any one of Claims 1 to 3, wherein said Rf1 locus is **characterized by** the presence of at least a nucleic acid of SEQ ID NO: 64 or a nucleic acid encoding an amino acid sequence having at least 95% identity, preferably 96%, 97%, 98%, 99% or 100% identity to SEQ ID NO:64.

5. The method according to any one of Claims 1 to 4, wherein the Rf3 locus is located within the chromosomal fragment between SNP markers cfn1249269 of SEQ ID NO:19 and BS00090770 of SEQ ID NO:42.

6. The method according to Claim 5, wherein said Rf3 locus is **characterized by** the presence of one or more of the following SNP restorer allele(s):
| SNP# | Marker Name | Marker SEQ ID | Restorer Allele |
|---|---|---|---|
| SNP17 | cfn1252000 | 17 | A |
| SNP18 | IWB14060* | 18 | G |
| SNP19 | cfn1249269 | 19 | G |
| SNP20 | 219K1_166464 | 20 | T |
| SNP21 | 219K1_158251 | 21 | G |
| SNP22 | 219K1_111446 | 22 | A |
| SNP23 | 219K1_110042 | 23 | T |
| SNP24 | 219K1_110005 | 24 | C |
| SNP25 | 219K1_107461 | 25 | A |
| SNP26 | 219K1_99688 | 26 | T |
| SNP27 | 219K1_37 | 27 | C |
| SNP28 | cfn1270524 | 28 | T |
| SNP29 | 136H5_3M5_7601 | 29 | T |
| SNP30 | cfn1288811 | 30 | G |
| SNP31 | 136H5_3M5_89176 | 31 | A |
| SNP32 | 136H5_3M5_89263 | 32 | T |
| SNP33 | 136H5_3M5_138211 | 33 | T |
| SNP34 | cfn0556874 | 34 | C |
| SNP35 | 136H5_3M5_64154 | 35 | C |
| SNP36 | 136H5_3M5_68807 | 36 | G |
| SNP37 | 136H5_3M5_77916 | 37 | A |
| SNP38 | cfn1246088 | 38 | A |
| SNP39 | cfn1287194 | 39 | G |
| SNP40 | cfn1258380 | 40 | A |
| SNP41 | IWB72107* | 41 | A |
| SNP42 | BS00090770 | 42 | T |
| SNP43 | cfn1239345 | 43 | A |
| SNP44 | RFL29_S2 | 44 | G |
| SNP 45 | RFL29_S4 | 45 | C |

7. The method according to any one of Claims 5 or 6, wherein said Rf3 locus is **characterized by** the presence of a nucleic acid encoding an amino acid sequence having at least 95% identity, preferably at least 96%, 97%, 98%, 99% or 100% identity to an amino acid selected from the group consisting of SEQ ID NO: 70, SEQ ID NO: 71 and SEQ ID NO:72.

8. The method according to any one of Claims 1 to 7, wherein said Rf4s locus is located within the chromosomal interval between SNP markers TaConting158085_61_BS0001 1513 of SEQ ID NO:46 and cfn0864865 of SEQ ID NO:47.

9. The method according to any one of Claims 1 to 8, wherein said Rf4s locus comprises any *Ae. Speltoides* SNP on the short arm of the chromosome 6B on the area ranging from 0 to 32 334 597 bases according to IWGSC V1 reference, preferably from the area ranging from 0 to 29 782 272 bases according to IWGSC V1 reference.

10. The method of Claim 8 or 9, wherein said Rf4s locus is **characterized by** the presence of one or more of the following SNP allele(s):
| SNP# | Marker Name | Marker SEQ ID | Restorer Allele |
|---|---|---|---|
| SNP46 | TaContigl 58085_61_BS00011513 | 46 | T |
| SNP47 | cfn0864865 | 47 | G |
| SNP48 | EXCALIBUR_C96134_152 | 48 | C |
| SNP49 | cfn3133296 | 49 | G |
| SNP50 | LWE1_chr6B_485210_Rf4S | 50 | T |
| SNP51 | LWE1_chr6B_11287944_Rf4S | 51 | G |
| SNP52 | LWE1_chr6B_19775886_Rf4S | 52 | G |
| SNP53 | LWE1_chr6B_28157776_Rf4S | 53 | C |

11. The method according to any one of Claims 1 to 10, wherein the plant also comprises Rf7 and/or 6R locus.

12. The method according to Claim 11, wherein the Rf7 locus is located at most 10 cM from SNP marker cfn0919993 of SEQ ID NO:55.

13. The method according to Claims 11 or 12, wherein said Rf7 locus is **characterized by** the presence of one or more of the following restorer SNP allele(s):
| SNP# | Marker Name | Marker SEQ ID | Restorer Allele |
|---|---|---|---|
| SNP54 | cfn0917304 | 54 | T |
| SNP55 | cfn0919993 | 55 | G |
| SNP56 | cfn0920459 | 56 | C |
| SNP57 | cfn0915987 | 57 | G |
| SNP58 | cfn0920253 | 58 | A |
| SNP59 | cfn0448874 | 59 | T |
| SNP60 | cfn0923814 | 60 | C |
| SNP61 | cfn0924180 | 61 | G |
| SNP62 | cfn0919484 | 62 | G |
| SNP64 | LWE1_chr7B _658281643_Rf7 | 263 | G |
| SNP65 | LWE1_chr7B_711539100_Rf7 | 264 | A |

14. The method according to any one of Claims 11 to 13, wherein the Rf7 locus is **characterized by** the haplotype "T", "G", "C", "G", "A", "T", "C", "G", "G", "G" and "A" and of the SNP54 to SNP62 and SNP64 to SNP65 restorer alleles respectively as described in the Table of Claim 13.

15. The method according to any one of Claims 11 to 14, wherein it further includes the 6R locus, said 6R locus being located on chromosome 6R and within the chromosomal interval between 48.9 cM to 114.8 cM, optionally, said 6R locus is **characterized by** the presence of the following restorer SNP allele:
| SNP# | Marker Name | Marker SEQ ID | Restorer Allele |
|---|---|---|---|
| SNP63 | RFL46_S2 | 63 | A |
